# EUROPEAN PATENT APPLICATION

(11) **EP 2 409 966 A1**
(43) Date of publication of application: **25.01.2012**
(21) Application number: 10753161.8
(22) Date of filing: 18.03.2010
(51) Int. Cl.: C07C 233/36, C07H 5/04

(54) **AMINOCYCLITOL COMPOUNDS, PROCEDURE FOR THE OBTAINMENT AND USES**

(30) Priority: 20.03.2009 ES 200900755
(71) Applicant: Consejo Superior de Investigaciones Cientificas (CSIC), 28006 Madrid (ES); Universidad Autónoma de Barcelona, 08193 Bellaterra (ES); Universitat De Barcelona, 08007 Barcelona (ES)
(72) Inventor: LLEBARIA SOLDEVILLA, Amadeo, E-08034 Barcelona (ES); BEDIA GIRBÉS, Carmen, E-08034 Barcelona (ES); HARRAK SERIFI, Youssef, E-08034 Barcelona (ES); CASTAÑO GARCÍA, Angel Raul, E-08193 Bellaterra (Barcelona) (ES); BARRA QUAGLIA, Carolina Mercedes, E-08193 Bellaterra (Barcelona) (ES); DELGADO CIRILO, Antonio, E-08007 Barcelona (ES)
(74) Representative: Ungria Lopez, Javier
(86) International application number: PCT/ES2010/070164
(87) International publication number: WO 2010/106215

(57) **Abstract**

Aminocyclitol compounds, process for obtaining them and uses

Aminocyclitol compounds and uses thereof as pharmaceutical compositions for the treatment of diseases associated with alterations in iNKT cells, more specifically autoimmune diseases, cancer, infections caused by pathogenic microorganisms or inflammatory diseases. Furthermore, the invention relates to the process for obtaining said compounds,

## Description

The present invention relates to the compounds of general formula (I) and uses thereof as pharmaceutical compositions for the treatment of diseases associated with alterations in iNKT cells. Furthermore, the invention relates to the process for obtaining said compounds. Thus, the invention may be included within the chemical and/or pharmaceutical field.

### STATE OF THE ART

Natural killer T cells, NKT, are a class of lymphocytes regulating a wide range of immune responses. A subclass of NKT cells, invariant NKT cells (iNKT), recognize glycolipidic type antigens presented by MHC CD1d proteins, and participating in various immune responses by promoting the secretion of cytokines. A role of these cells has been described in the regulation of autoimmunity, tumor response, microbial infections and in the pathogenesis of inflammatory processes such as asthma.

The synthetic glycolipid α-galactosylceramide (α-Galcer) was the first antigen known presented by CD1d that stimulates the invariant T cells receptor (TCR), expressed by iNKT cells. α-Galcer is an optimized structural analog of antitumor compounds isolated from sea sponges that contains a galactose bound to the phytoceramide primary hydroxyl in alpha configuration, and that is found acylated in the nitrogen with long chain fatty acids. Since its discovery, α-GalCer has been the antigen prototype for stimulating iNKT cells, although other glycolipids that act as antigens presented by CD1 proteins have also been identified. The exceptional potency of α-Galcer in the stimulation of NKT cells raises a series of biological responses, derived from the simultaneous release of Th1- and Th2-type cytokines, exerting opposite cellular effects and determining an anergy phase before restoring the homeostatic balance, limiting the efficacy of α-Galcer as immunomodulator.

On the other side, the structural determinants of the glycolipid interaction with the presenting protein CD1d are known, as well as the crystalline structure of the complex of the α-Galcer bound to the proteins CD1d and TCR. These structural studies show that the ceramide binds to CD1d in two hydrophobic pockets wherein the lipid chains are inserted, while a net of hydrogen bonds blocks the position of the galactose, projecting from the binding site, and is presented to the TCR for its recognition, wherein the hydroxyl groups in the glycolipid play a major role.

The extraordinary potency of the stimulation of NKT cells by α-Galcer is not free of problems, fact that has promoted the design and synthesis of similar compounds with the purpose of enhancing its biological properties, even at the expense of obtaining less potent compounds, and mainly directed to the modulation of the Th1/Th2 ratio in the production of induced cytokines.

Several approaches have been described for the design of analog compounds of α-Galcer, mainly in connection with the variations in the length or nature of the lipids (cfr. C. McCarthy, et al., J Exp Med 2007, 204, 1131; M. Trappeniers, et al., ChemMedChem 2008, 3, 1061; K. Fuhshuku, et al., Bioorg Med Chem 2008, 16, 950; T. Tashiro, et al., Bioorg Med Chem 2008, 16, 8896) or the substitution of galactose by other mono- or polysaccharides (cfr. T. Kawano et al., Science 1997, 278, 1626), and various other related substitutions (cfr. US2007238871A1). Some of the α-GalCer analogs with linear non-glycosidic groups are able to activate iNKT cells and induce immunological responses, which demonstrates that it is possible to modulate the glycolipid effects after the introduction of structural modifications in the galactose ring of α-Galcer (cfr. J. D. Silk et al., J Immunol 2008, 180, 6452; R. W. Franck, Acc Chem Res 2006, 39, 692).

In view of the above, it would be appropriate the discovery of novel glycolipid type compounds, or either known glycolipid analogs, such as α-GalCer, allowing the modulation of the cell-mediated immune response, and being useful for the development of medicaments, adjuvants, vaccines and any immunotherapy designed for stimulating NKT cells.

### DESCRIPTION OF THE INVENTION

The present invention provides glycolipid analog compounds capable of stimulating and activating NKT cells with preferential production of Th2-type cytokines. The structure of these compounds consists in the presence of a polyhydroxylated cyclohexylamine bound to the primary hydroxyl of a ceramide type lipid by a secondary amine bond.

A first aspect of the present invention relates to the compounds of general formula (I), or its isomers, salts and/or solvates thereof (hereafter compounds of the invention): 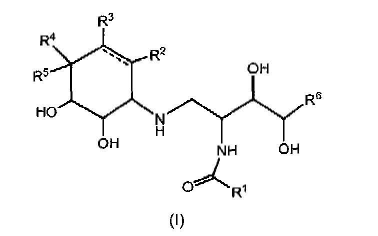 wherein: R¹ is a (C₅-C₃₅)alkyl group, substituted or unsubstituted.
R², R³, R⁴ and R⁵ are the same or different from each other, and are selected from the list comprising hydrogen (H), hydroxyl (OH), alkoxyl (ORₐ) or (C₁-C₆)alkyl, substituted or unsubstituted;
R⁶ is selected from the list comprising the following groups, substituted or unsubstituted, (C₅-C₃₅)alkyl, aryl, cycloalkyl or heterocycle; and
---- represents the existence or not of a double bond.

The term "alkyl" relates to in the present invention, in the case of R¹ and/or R⁶, aliphatic chains, straight or branched, having 6 to 35 carbon atoms. In the case of R², R³, R⁴ and/or R⁵, the term alkyl relates to aliphatic chains, straight or branched, having 1 to 6 carbon atoms, for example, methyl, ethyl, n-propyl, *i*-propyl, *n*-butyl, *tert*-butyl, *sec*-butyl, *n*-pentyl, *n*-hexyl, while having preferably 1 to 3 carbon atoms. The alkyl groups can be optionally substituted by one or more substituents such as halogen, hydroxyl, azide, carboxylic acid or a group, substituted or unsubstituted, selected from: aryl, hydroxyl, amine, amide, ester, carboxylic acid, ether, thiol, acylamine or carboxamide, When the alkyl group is substituted by an aryl is described as "arylalkyl", as for example, but not limited to, in the case of a benzyl group.

The term "alkoxyl" relates to, in the present invention, a group of formula -ORₐ, wherein Rₐ is a (C₁-C₈)alkyl, as for example, and not limited to, methoxyl, ethoxyl or propoxyl. Preferably the alkoxyl is a methoxyl.

The term "aryl" relates to, in the present invention, an aromatic carbocyclic chain, having 6 to 18 carbon atoms, which may be of a single or multiple rings, in this last case with separated and/or fused rings. May be given as examples of aryl group, but not limited to, the groups: phenyl, naphthyl, indenyl, etc. Preferably the aryl group is a phenyl.

"Cycloalkyl" relates to a stable monocyclic or bicyclic radical of 3 to 10 members, being saturated or partially saturated, and consisting only in carbon and hydrogen atoms, such as cyclopenthyl, cyclohexyl or adamanthyl.

The term "heterocyclic" relates to, in the present invention, a stable monocyclic or bicyclic radical of 3 to 10 members, being unsaturated, saturated or partially saturated, and consisting in carbon atoms and at least one heteroatom selected from the group consisting in nitrogen, oxygen or sulphur. Preferably the heteroatom is nitrogen, and more preferably the cycle is a 5- or 6-membered ring. Examples of heterocycloalkyls may be, not limited to: piperidine, piperazine, purine, pirazoline or pyrrolidine.

In a preferred embodiment of the compounds of the invention, R¹ is a (C₇-C₂₅)alkyl group.

In another preferred embodiment of the compounds of the invention, R², R³, R⁴ and/or R⁵ are hydroxyl. More preferably, R⁴ is hydroxyl and/or R⁵ is hydrogen and/or R³ is hydroxyl or hydroxyalkyl, more preferably said hydroxyalkyl (R³) is an hydroxymethyl and/or R² is hydrogen, an hydroxyl or alkoxyl group, in which case said alkoxyl is more preferably methoxyl.

In another preferred embodiment of the compounds of the invention, R⁶ is a (C₁₀-G₂₀)alkyl group.

In a more preferred embodiment the compounds of the invention are selected from the list comprising:
(2*S*,3*S*,4*R*)-2-octanamide-1-(1*'rs,*2'*RS,*3'*SR,*4'*SR,*5'*RS,*6'*SR*)-2',3',4',5',6'-pentahydroxycyclohexylaminooctadecane-3,4-diol;
(2*S*,3*S*,4*R*)-2-Hexacosanamide-1-(1'*rs,*2'*RS*,3'*SR*,4'*sr*,5'*RS,*6'*SR*)-2',3',4',5',6'-pentahydroxycyclohexylaminooctadecane-3,4-diol,
(2*S*,3*S*,4*R*)-2-octanamide-1-(1'*R*,2'*S*,3'*R*,4'*S*,5'*S*,6'*S*)-2',3',4',5',6'-pentahydroxycyclohexylaminooctadecane-3,4-diol;
(2*S*,3*S*,4*R*)-2-Hexacosanamide-1-(1'*R*,2'*S*,3'*R*,4'*S*,5'*S*,6'*S*)-2,3,4,5,6-pentahydroxycyclohexylaminooctadecane-3,4-diol;
(2*S*,3*S*,4*R*)-2-hexacosanamide-1-(1'*S*,2'*S*,3'*R*,4'*R*,5'*S*)-2,3,4,5,-tetrahydroxycyclohexylaminooctadecane-3,4-diol;
(2*S*,3*S*,4*R*)-2-hexacosanamide-1-(1'*R*,2'*S*,3'*R*,4'*R*,5'*S*)-2,3,4,5,-tetrahydroxycyclohexylaminooctadecane-3,4-diol;
(2*S*,3*S*,4*R*)-2-hexacosanamide-1-(1'*S*,2'*S*,3'*R*,4'*R*,5'*S*,6'*S*)-2,3,4,5,-tetrahydroxy-6-methoxycyclohexylaminooctadecane-3,4-diol;
(2*S*,3*S*,4*R*)-2-Hexacosanamide-1-(1'*S*,4'*S*,5'*S*,6'*S*)-4',5',6'-trihydroxycyclohexenylaminooctadecane-3,4-diol;
(2*S,*3*S*,4*R*)-2-Hexacosanamide-1-(1'*S*,4'*S*,5'*S*,6'*S*)-4',5',6'-trihydroxycyclohexylaminooctadecane-3,4-diol;
(2*S*,3*S*,4*R*)-2-hexacosanamide-1-((1'*S*,2'*S*,3'*S*,4'*S*,5'*S*,6'*R*)-5-hydroxymethyl-2,3,4,6,-tetrahydroxycyclohexylamino)octadecane-3,4-diol;
(2*S*,3*S*,4*R*)-2-hexacosanamide-1-((1'*S*,2'*S*,3'*S*,4'*S*,5'*R*)-5-hydroxymethyl-2,3,4,-trihydroxycyclohexylamino)octadecane-3,4-diol;
(2*S*,3*S*,4*R*)-2-hexacosanamide-1-((1'*R*,2'*S*,3'*S*,4'*S*,5'*R*)-5-hydroxymethyl-2,3,4,-trihydroxycyclohexylamino)octadecane-3,4-diol; or any salt thereof, preferably hydrochlorides.

Another aspect of the present invention relates to the compounds of general formula (I) for use in the preparation of a medicament or pharmaceutical composition, preferably for the treatment and/or prevention of autoimmune diseases, cancer, infections caused by pathogenic microorganisms, inflammatory diseases, and, in general, any disease for which treatment or prevention is likely to benefit from the biological activities shown by the products described in the present invention through the stimulation of iNTK cells, or either to a salt, derivative or solvate thereof, or either to a pharmaceutically acceptable prodrug thereof.

The compounds of general formula (I) can also be used for the preparation of vaccines or vaccination adjuvants, or other methods of activating the immune system response known by the skilled in the art.

The infectious diseases caused by pathogenic microorganisms may be viral infections as, e.g., flu, AIDS or hepatitis; bacterial infections as, e.g. chlamydiosis, tuberculosis, streptococcosis, or pseudomoniasis; or parasitic infections as, e.g. leishmaniasis, malaria or trypanosomiasis.

The autoimmune and/or inflammatory diseases may be selected from the list comprising, not limited to, systemic lupus erythematosus, type 1 diabetes mellitus, multiple sclerosis, Sjogren syndrome, rheumatoid arthritis, asthma, chronic obstructive pulmonary disease (COPD), chronic colitis or several allergies,

The term "cancer" or "cancerous", as used in the present description, relates to an alteration of tumor cells that are capable of invading tissues or producing metastases in distant places from the primary tumor. Examples of cancer are, not limited to: breast cancer, gynecological cancer, colon cancer, prostate cancer, skin cancer, hepatocelular cancer, lung cancer, esophagus cancer, gastric cancer, pancreas cancer, bladder cancer, liver cancer, urinary tract cancer, thyroid cancer, renal cancer, melanoma, brain cancer, sarcoma, lymphoma or leukemia.

The compounds of the present invention represented by formula (I) may include isomers, depending on the presence of multiple bonds (for example, Z, E), including optical isomers or enantiomers, depending on the presence of chiral centers. The individual isomers, enantiomers or diastereoisomers and mixtures thereof fall within the scope of the present invention, i. e., the term isomer also relates to any mixture of isomers, as diastereomers, racemics, etc., even to its optically active isomers or mixtures in different ratios thereof. The individual enantiomers or diastereomers, as well as the mixtures thereof, can be separated by standard techniques.

As used herein, the term "derivative" includes both pharmaceutically acceptable compounds, i. e., derivatives of the compound of formula (I) that can be used in the preparation of a medicament, and pharmaceutically inacceptable derivatives, since these can be useful in the preparation of pharmaceutically acceptable derivatives, The nature of the pharmaceutically acceptable derivative is not critical as long as it is pharmaceutically acceptable.

Likewise, within the scope of this invention are found the prodrugs of the compounds of formula (I). The term "prodrug" as used herein includes any compound derived from a compound of formula (I) -for example, and not limited to: esters (including carboxylic acids esters, amino acid esters, phosphate esters, metallic salts sulfonate esters, etc.), carbamates, amides, etc.- that when administered to an individual can be converted directly or indirectly in said compound of formula (I) in the mentioned individual. Advantageously, said derivative is a compound that increases the bioavailability of the compound of formula (I) when it is administered to an individual, or that enhances the release of the compound of formula (I) in a biological compartment. The nature of said derivative is not critical as long as it can be administered to an individual and provides the compound of formula (I) in a biological compartment of an individual. The preparation of said prodrug may be carried out by standard methods known by the skilled in the art.

The compounds of the invention can be in crystalline form as free compounds or as solvates. In this sense, the term "solvate", as used herein, includes both pharmaceutically acceptable solvates, i. e., solvates of the compound of formula (I) that can be used in the preparation of a medicament, and pharmaceutically inacceptable solvates, which can be useful in the preparation of pharmaceutically acceptable solvates or salts, The nature of the pharmaceutically acceptable solvate is not critical as long as it is pharmaceutically acceptable. In a particular embodiment, the solvate is an hydrate. The solvates can be obtained by solvation standard methods known by the skilled in the art.

For its application in therapy, the compounds of formula (I), its derivatives isomers, salts, prodrugs or solvates will be found, preferably, in a pharmaceutically acceptable or substantially pure form, i. e., having a pharmaceutically acceptable level of purity excluding the normal pharmaceutical additives such as diluents and carriers, and not including material considered toxic at normal dosing levels. The levels of purity for the active ingredient are preferably higher than 50%, more preferably higher than 70%, and even more preferably higher than 90%. In a preferred embodiment, they are higher than 95% of the compound of formula (I), or its salts, solvates or prodrugs.

The compounds of the present invention of formula (I) can be obtained or produced through a chemical synthetic route, or obtained from a natural matter of different origin.

Another aspect of the present invention relates to a process for obtaining the compounds of the invention of formula (I) or an isomer, its salts and/or solvate thereof, comprising the following steps that are outlined in scheme 1:
A) preparation of aminocyclitols of general formula (II), by:
   A1) reduction of cyclohexyl or cyclohexenyl azides (general formula (III)), wherein the hydroxyl substituents have been blocked with a protecting group (PG), preferably benzyl, or
   A2) by substitution of cyclohexyl or cyclohexenyl halides or sulfonates (general formula (IV)) with ammonia or other amines, wherein the hydroxyl substituents have been blocked with a protecting group (PG), preferably benzyl;
B) activation of phytosphingosine or analog molecules by the preparation of:
   B1) aldehydes (VI), wherein the hydroxyl substituents have been blocked with a protecting group, preferably benzyl, or
   B2) by the formation of aziridines (V), wherein the hydroxyl substituents of the diol have been blocked with a protecting group, preferably benzyl o isopropylidene.
C) coupling of the aminocyclitols obtained according to step (A) with:
   C1) the aldehydes obtained in step (B1) by reductive amination; or
   C2) nucleophilic attack on the phytosphingosine-derived aziridines, obtained in the step (B2); and
D) acylation of the amine present in the phytosphingosine with carboxylic acids or its derivatives, followed by the removal of the protecting groups.

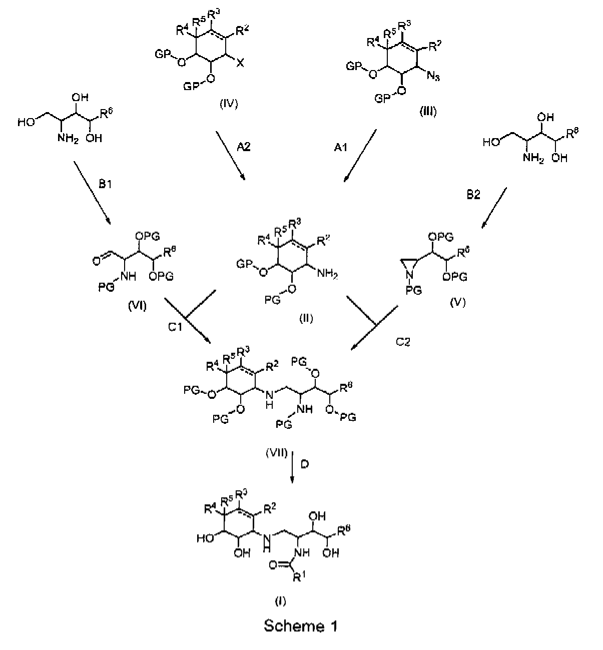

Wherein: R¹, R², R³, R⁴, R⁵ and R⁶ are as described above; X is an halide or sulfonate; and PG is a protecting group.

Another further aspect of the present invention relates to a pharmaceutical composition useful for the treatment and/or prevention of diseases through the stimulation of iNKT cells, hereinafter pharmaceutical composition of the invention, comprising a compound or mixture of compounds of formula (I), in a therapeutically effective amount, or a pharmaceutically acceptable salt, prodrug, solvate, derivative or stereoisomer thereof, along with a pharmaceutically acceptable carrier, adjuvant or vehicle, for administration to a patient.

The pharmaceutically acceptable adjuvants and vehicles that can be used in said compositions are the adjuvants and vehicles known by the skilled in the art and usually used in the preparation of therapeutic compositions.

In the sense used in this description, the expression "therapeutically effective amount" relates to the amount of agent or compound capable of developing the therapeutic action determined by its pharmacological properties, calculated for producing the intended effect and, in general, it would be determined, among other causes, by the compounds' own characteristics, including the age, the patient's condition, the severity of the disturbance or disorder, and the route and frequency of administration.

The compounds described in the present invention, its derivatives, salts, prodrugs and/or solvates, as well as the pharmaceutical compositions containing thereof, can be used along with other additional drugs, or active ingredients, for providing a combination therapy. Said additional drugs can be part of the same pharmaceutical composition or, alternatively, can be provided in the form of a discrete composition for a simultaneous or not administration to that of the pharmaceutical composition comprising a compound of formula (I), or a prodrug, solvate, derivative or its salts.

In another particular embodiment, said therapeutic composition is prepared in the form of a solid form or aqueous suspension, in a pharmaceutically acceptable diluent. The therapeutic composition provided by this invention can be administered through any suitable route of administration, for which said composition will be formulated in the pharmaceutical form suited to the route of administration chosen. In a particular embodiment, the administration of the therapeutic composition provided by this invention is accomplished orally, topically, rectally or parenterally (including subcutaneously, intraperitoneally, intradermically, intramuscularly, intravenously, etc.).

The use of the compounds of the invention is compatible with its use in protocols in which the compounds of formula (I), or mixtures thereof, are used *per se* or in combinations with other treatments or any medical procedure.

Throughout the description and the claims the word "comprises" and its variants are not intended to preclude other technical characteristics, additives, components or steps. For the skilled in the art, other objects, advantages and characteristics will become apparent in part from the description and in part from the practice of the invention. The following examples and figures are provided by way of illustration, and are not intended to be limiting of the present invention.

### DESCRIPTION OF THE FIGURES

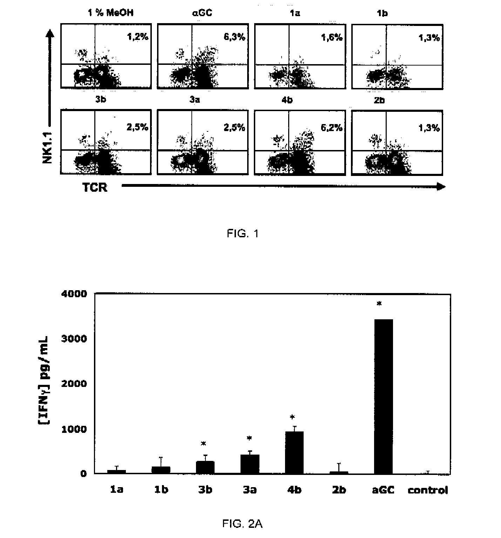

Fig. 1.- Shows the analysis by flow cytometry of mouse spleen cells incubated with different compounds for 5.5 days. The compounds were assayed at a final concentration of 1 µg/mL in complete medium with 1% of methanol (MeOH), while the αGalcer (αGC) was used at 0.2 µg/mL. The cell cultures were labeled with anti-NK1-PE and anti-TCR-FITC specific antibodies. The percentages of NKT cells (double positive cells) were calculated within the population of electronically selected T cells.

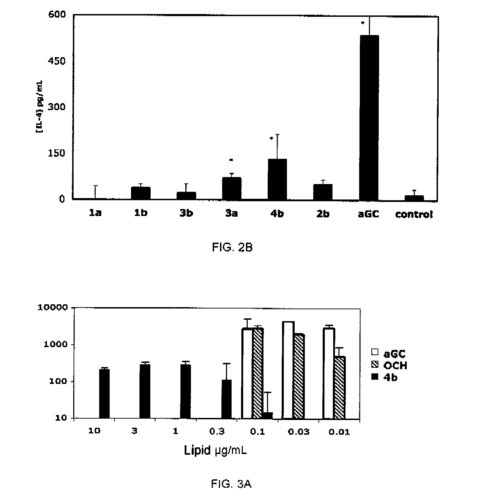

Fig. 2.- Shows the ability of aminocyclitol type compounds to induce cytokines for stimulating NKT cells. Spleen cells derived from 3 B6 mice were combined and cultured in tetraplicate with 1 µg/mL of aminocyclitols or 0.1µg/mL of α-GalCer (αGC), and the IFNγ (Fig. 2A) and IL-4 (Fig. 2B) cytokines present in the supernatants of the cultures at four days were determined by ELISA (2 determinations). The data represent the mean ± SD of a representative experiment. * Indicates statistically significant results against control cultures without compound to be assayed.

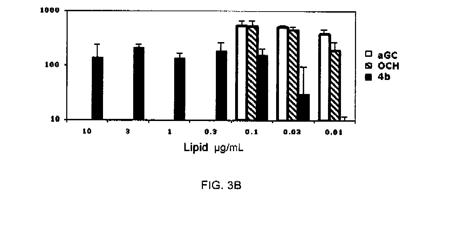

Fig. 3.- Shows how the compound 4b induces Th1- and Th2-type cytokines produced by NKT cells. Quantifying of the production of IFNγ (Fig. 3A) and IL-4 (Fig. 3B) after the stimulation *in vitro* of cultures of mouse spleen cells containing lipidic agonists of iNKT cells in a dose-response assay. The data show the mean + SD of a representative experiment with 2 mice, duplicate cultures of each mouse at the indicated concentrations of agonist, and 3 ELISA determinations for each culture supernatant.

### EXAMPLES

In the following the invention will be illustrated by some assays performed by the inventors that develop the selection process of the compounds of the invention.

### Preparation of phytosphingosine-derived amides

### Example 1:

### N-((2S,3S,4R)-3,4-bis(benzyloxy)-1-hydroxyoctadecan-2-yl)octanamide

To a solution of 249 mg (0.5 mmol) of 3,4 di-O-benzylphytosphingosine (C. Xia et al. Bioorganic & Medicinal Chemistry Letters 2006, 16, 2195-2199) in THF (tetrahydrofuran) (10 mL), octanoic acid (143 mg, 1.1 mmol) and the *N*-(3-Dimethylaminopropyl)-*N'*-ethylcarbodiimide hydrochloride (EDC, 1.1 mmol) are added, and the reaction mixture is stirred at reflux for 3 h. The mixture is cooled to room temperature and an NH₄Cl saturated solution is added. It is extracted with Et₂O (ethyl ether) and dried with anhydrous Na₂SO₄. The solid is filtered and the solvents are evaporated to give a crude that is purified in silica gel chromatography using a mixture (ethyl acetate/hexane 1:10 to 1:5 v:v). The product (249 mg) is obtained in the form of an oil with an 81% yield.

[α]_{D} = -32.3 (CDCl₃, c, 1.0); IR (neat, cm⁻¹); 3305, 2929, 1654, 1540, 1070; ¹H-NMR (CDCl₃, 500 MHz); δ 7.35 (m, 10H); 6.06 (d, *J* = 8.5, 1H); 4.73 (d, *J* = 12, 1H); 4.67 (d, *J* = 12, 1H); 4.62 (d, *J =* 12, 1H); 4.46 (d, *J* = 12, 1H); 4.15 (m, 1H); 4.00 (dd, *J* = 11.5, 3, 1H); 3.70 (m, 2H); 3,62 (dd, *J* = 11.5, 4.5 1H); 3.05 (br, 1H); 2.00 (m, 2H), 1.77 -1.41 (m, 6H); 1.26 (m, 30 H); 0.88 (t, *J* = 7, 6H). ¹³C-NMR (CDCl₃, 100 MHz): δ 172.8, 138.1, 137.7, 128.6-127.7 (10 C), 82.1, 79.0, 73.0, 72.8, 62.9, 50.5, 36.6, 31.8, 31.6, 30.7, 29.6-29.0 (11 C), 25.9, 25.6, 22.6 (2C), 14.0 (2C). HRMS (High resolution mass spectrometry). Calculated for C₄₀H₆₅NO₄ (M+H⁺): 624.4992. Found: 624.5015.

### Example 2:

### N-((2S,3S,4R)-3,4-bis(benzyloxy)-1-hydroxyoctadecan-2-yl)hexacasanamide

In an way analogous to that described in example 1, the amide is obtained from 302 mg of hexacosanoic acid, 251 mg of 3,4 di-O-benzylphytosphingosine and 215 mg of EDC to give a colorless solid (83%).

Pf: 102-103; [α]_{D} = -27.2 (CDCl₃, c, 1.0); IR (neat, cm⁻¹): 3310, 2945, 1659,1533,1063; ¹H-NMR (CDCl₃, 100 MHz); δ 7.34 (m, 10 H); 6.03 (d, *J* = 8, 1H); 4.72 (d, *J* = 11.5, 1H); 4.66 (d, *J* = 11.5, 1H); 4.61 (d, *J* = 11.5, 1H); 4.45 (d, *J* = 11.5, 1H); 4.13(m, 1H); 3.99 (dd, *J* = 11.5_{.} 31H); 3.70 (m, 2H); 3.61 (dd, *J* = 11.5, 4.4, 1H); 3.05 (br, 1H); 2.00 (m, 2H),1.76-1,40 (m, 6H); 1.27 (m, 66H); 0.87 (t, *J* = 7, 6H). ¹³C-NMR (CDCl₃, 100 MHz): δ 172.8, 138.1, 137.8, 128.7-127,8 (10C), 82.3, 79.0, 73.0, 72.9, 63.0, 50.5, 36.7, 31.9 (2C), 30.8, 29.7-29.3 (29C), 26.0, 25.6, 22.7 (2C), 14,1 (2C). HRMS. Calculated for C₅₈H₁₀₁NO₄ (M+H⁺): 876.7609, Found: 876.7828.

### Preparation of aldehydes

### Example 3:

### N-((2S,3S,4R)-3,4-bis(benzyloxy)-1-oxooctadecan-2-yl)octanamide

According to a method described [M. Ocejo, et al Synlett 2005, 13, 2110-2112] 123 mg (0.2 mmol) of the *N*-((2*S*,3*S*,4*R*)-3,4-bis(benzyloxy)-1-hydroxyoctadecan-2-yl)octanamide prepared in example 1 are dissolved in ethyl acetate (AcOEt) (40 mL) and 224 mg (0.8 mmol) of o-iodoxybenzoic acid (IBX) are added at room temperature, It is heated to reflux for 3 h and then the mixture is brought to room temperature, diluted with hexane (40 mL) and the solid is filtered. The filtrate is evaporated to give 120 mg of the aldehyde of high purity with a yield next to 100%. This material is used immediately in the next reactions.

IR (film, cm⁻¹): 3383, 2903, 1708, 1497, 1041, 738. ¹H-NMR (CDCl₃, 500 MHz); δ: 9.67 (s, 1H), 7.30-7,28 (m, 10H); 6.06 (d, *J* = 7.5, 1H); 4.96 (dd, 7.5, *J* = 2.5, 1H); 4.61 (m, 2H); 4.54 (m, 2H); 3.94 (m, 1H); 3.62 (q, *J* = 5.5, 1H); 2.04 (m, 2H); 1.71 (m, 2H); 1.53 (m, 2H); 1.28 (m, 32H); 0.90 (t, *J* = 7, 6H); ¹³C-NMR (CDCl₃, 100 MHz): δ 198.0, 172.1, 137.3, 137.1, 128.5-127.8 (10C), 81.3, 76.6, 72.0, 71.5, 58.6, 36.8, 31.9, 30.3, 29.6-28.9 (13C), 25.5, 24.6, 22.8, 14.1(2C).

### Example 4:

### N-((2S,3S,4R)-3,4-bis(benzyloxy)-1-oxooctadecan-2-yl)hexacosanamide.

According to the procedure of example 3, the aldehyde is obtained by oxidation with IBX of the *N*-((2*S*,3*S*,4*R*)-3,4-bis(benzyloxy)-1-hydroxyoctadecan-2-yl)hexacosanamide obtained according to example 2,

IR (film, cm⁻¹): 3381, 2900, 1711, 1502, 1045, 740. ¹H-NMR (CDCl₃, 500 MHz); δ: 9.68 (s, 1H), 7.39-7.28 (m, 10H); 6.06 (d, *J* = 8, 1H); 4.96 (dd, *J* = 7.5, 2.5, 1H); 4.62 (d, *J* =11, 1H) 4.61 (d, *J* = 11, 1H); 4.54 (d, *J* = 11, 1H) 4.53 (d, *J* = 11, 1H); 3.94 (dd, *J* = 5.5, 2.5, 1H); 3.63 (q, *J* = 5.5, 1H); 2,04 (m, 2H), 1.72 (m, 2H); 1.53 (m, 2H); 1.28 (m, 68H); 0.90 (t, *J* = 7, 6H); ¹³C-NMR (CDCl₃, 100 MHz): δ 198.1, 173.5, 137.5, 137.4, 128.6-127.8 (10C), 81.5, 76.9, 72.1, 71.7, 58.7, 36.4, 31.9, 30.1, 29.7-29.2 (31C), 25.5, 24.7, 22.7, 14.1(2C).

### Reductive amination

**Example 5:**

### (2S,3S,4R)-(3,4-dibenzyloxy-1-(1'rs,2'RS,3'SR,4'SR,5'RS,6'SR)-2',3',4',5',6'-pentabenzyloxycyclohexylaminooctadecan-2-yl)octanamide

A solution of 158 mg (0.25 mmol) of (1'*rs*,2'*RS*,3'*SR*,4'*SR*,5'*RS*,6'*SR*)-2,3,4,5,6-pentakis(benzyloxy)cyclohexanamine [Serrano et al. J Org Chem 2005, 70, 7829] in methanol/CH₂Cl₂ (1:3, v/v) (4 mL) under an argon atmosphere is treated successively, and in this order, with sodium cyanoborohydride (3 equiv.), acetic acid (20 µL) and the N-((2*S*,3*S*,4*R*)-3,4-bis(benzyloxy)-1-oxooctadecan-2-yl)octanamide obtained according to the procedure of example 3. It is stirred for 18 h at room temperature, the mixture is treated with water (3 mL) and extracted with ethyl acetate (3 X 20 mL). The organic extracts are combined, washed with water and dried with anhydrous sodium sulfate. The solid is filtered and the volatiles are evaporated to give a crude that is purified by silica gel chromatography, eluting with mixtures of hexane/ethyl acetate (3:1 to 1:1, v/v), to give 89 mg, 0.07 mmol (29%) of the product in the form of a clear oil.

[α]_{D} = -6.0 (CDCl₃, c, 1.0); IR (neat, cm⁻¹): 3336, 2915, 2847,1642,1451, 1048; 1H-NMR (CDCl₃, 500 MHz); δ 7.35-7.20 (m, 35 H); 6.39 (d, *J* = 8.5, 1H); 4.96-4.83 (m, 7 H); 4.78 (d, *J* = 11,5, 1H); 4.74 (d, *J* = 11.5, 1H); 4.65 (d, *J* =, 11.5, 1H); 4.58 (d, *J* = 11.5, 1H); 4.54 (d, *J* = 11.5, 1H); 4.50 (d, *J* = 11,5, 1H); 4.40 (d, J=11.5, 1H); 4.08 (m, 1H); 3.71 (dd, *J* = 5. 4, 1H); 3.57 (m, 3H); 3.45 (m, 1H); 3.24 (m, 3H); 2.95 (dd, *J* = 13, 4.5, 1H); 2.58 (t, *J* = 10, 1H); 1.75 (m, 2H), 1.58 (m, 2H); 1.40 (m, 4H); 1.28 (m, 30H); 0.90 (t, *J* = 7, 3H); 0.88 (t, *J* = 7, 3H). ¹³C-NMR (CDCl₃, 100 MHz): δ 172.6, 138.6, 138.5, 138.3, 138.2 (2C), 138.1 (2C), 128,4-127.1 (35C), 84.1, 83.9, 83.0, 82.4, 82.1, 80.1, 75.7 (2C), 75.6, 75.4, 74.5, 73.4, 71.3, 62.4, 49.9, 48.7, 36.3, 31.8, 30.0; 29.8-29.3 (12C), 25.7,25.6,22.6 (2C), 14.1, 14,0. HRMS. Calculated for C₈₁H₁₀₈N₂O₈ (M+H⁺): 1235.8027. Found: 1235.8049.

### Example 6:

### (2S,3S,4R)-(3,4-dibenzyloxy-1-(1'rs,2'RS,3'SR,4'SR,5'RS,6'SR)-2',3',4',5',6'-pentabenzyloxycyclohexylaminooctadecan-2-yl)hexacosanamide

By a procedure analogous to that described in example 5, the amide is obtained from (1s,2R,3S,4r,5R,6S)-2.3.4,5.6. pentakis(benzyloxy)cyclohexanamine (cfr. Serrano et al. J Org Chem 2005, 70, 7829) and *N*-((2*S*,3*S*,4*R*)-3,4-bis(benzyloxy)-1-oxooctadecan-2-yl)hexacosanamide by reductive amination with a 28% yield.

Oil, [α]_{D} = -6.3 (CDCl₃, c, 1.0); IR (film, cm⁻¹): 3513, 2933, 2855, 1719, 1450, 1074; ¹H-NMR (CDCl₃, 500 MHz); δ 7.39-7.22 (m, 35 H); 6.42 (d, *J* = 8.8, 1H); 4.95-4.82 (m, 7H); 4.79 (d, *J* =11.5. 1H); 4.73 (d, *J* =, 11.5, 1H); 4.63 (d, *J* =, 11.5, 1H); 4.59 (d, *J* =, 11.5, 1H); 4.53 (d, *J* =, 11.5, 1H); 4.49 (d, *J* =, 11.5, 1H); 4.40 (d, *J* = 11.5, 1H); 4.08 (m, 1H); 3.71 (dd, *J* = 5, 4, 1H); 3.57 (m, 3H); 3.46 (m, 1H); 3.25 (m, 3H); 2.96 (dd, *J* = 13, 4.5, 1H); 2.58 (t, *J* = 10, 1H); 1.74 (m, 2H), 1.58 (m, 2H); 1.40 (m, 4H); 1.28 (m, 66H); 0.90 (t, *J* = 7, 6H); 13C-NMR (CDCl₃, 100 MHz): δ 172.8, 138.5 (2C), 138.3, 138.2 (4C), 128.4-127.1 (35C), 84.1, 83.9, 83.0, 82.3, 82.0, 80.0, 75.8 (2C), 75.6, 75.4, 74.5, 73.4, 71.3, 62.3, 49.4, 48.7, 48.5, 36.3, 31.9, 31.7; 29.9-29,0 (30C), 25.7, 25.6, 22.6, 22.5, 14.1, 14.0. HRMS. Calculated for C₉₉H₁₄₂N₂O₈ (M+H⁺): 1488.0844. Found: 1488.0773.

### Example 7:

### (2S,3S,4R)-(3,4-dibenzyloxy-1-(1'R,2'S,3'R,4'S,5'S,6'S)-2',3',4',5',6'-pentabenzyloxycyclohexylaminooctadecan-2-yl)hexacosanamide

According to a procedure described in racemic series (Serrano et al, J Org Chem 2005, 70, 7829) from (+)-tetra-O-benzylconduritol B epoxide (cfr. González-Bulnes et al., Carbohydr Res 2007, 342, 1947-52) is prepared the compound (1*R*,2*S*,3*R*,4*S*,5*S*,6*S*)-2,3,4,5,6-pentahydroxycyclohexylamine, that is reacted according to the reductive amination procedure described in example 5 with the intermediate aldehyde *N*-((2*S*,3*S*,4*R*)-3,4-bis(benzyloxy)-1-oxooctadecan-2-yl)hexacosanamide to give the required compound with a 40% yield.

Oil, [α]_{D} = +1.6 (CDCl₃, c, 1.0); IR (film, cm⁻¹): 3431, 2916, 2847, 1653, 14940,1061; ¹H-NMR (CDCl₃, 500 MHz); δ 7.38-7.28 (m, 30 H); 6.16 (d, *J* = 8, 1H); 4.89-4.56 (m, 11H); 4.44 (d, *J* = 12, 1H); 4.24 (m, 1H); 4,01 (m, 1H); 3.98 (dd, *J* = 9, 4, 1H); 3.81 (m, 3H); 3.73 (t, *J* = 4, 1H); 3.56 (dd, *J* = 11, 5, 1H); 3.16 (t, *J* = 4, 1H); 2.94 (dd, *J* = 11,4, 1H); 2.61 (dd, *J* = 11, 5, 1H); 2.00 (m, 2H), 1.64 (m, 2H); 1.57 (m, 2H); 1.28 (m, 68H); 0.91 (t, *J* = 7, 3H); 0.90 (t, *J* = 7, 3H). ¹³C-NMR (CDCl₃, 100 MHz): δ 172.3, 138.8, 138.7, 138.3(2C), 138.2, 137.9, 128.4-127.3 (30C), 82.4, 81.9, 81.5, 80.4, 79.1, 79.0, 75.9, 75.6, 73.8, 73.1, 72.3, 71.4, 67.3, 58.2, 48.5, 48.4, 36.7, 31.8, 30.2, 29.7-29.3 (31 C), 25.6, 25.4, 22.6, 14.0 (2C). HRMS. Calculated for C₉₂H₁₃₆N₂O₈ (M+H⁺): 1398.0375. Found: 1398.0386.

### Example 8:

### (2S,3S,4R)-(3,4-dibenxyloxy-1-(1'R,2'S,3'R,4'S,5'S,6'S)-2',3',4',5',6'-pentabenzyloxycyclohexylamino)octadecan-2-yl)octanamide

It is prepared by reductive amination according to example 7 from (1*R*,2*S*,3*R*,4*S*,5*S*,6*S*)-2,3,4,5,6-pentahydroxycyclohexylamine and the intermediate aldehyde *N*-((2*S*,3*S*,4*R*)-3,4-bis(benzyloxy)-1-oxooctadecan-2-yl)hexacosanamide with a 38% yield.

Oil. [α]_{D} = +2.0 (CDCl₃, c, 1.0); IR (film, cm⁻¹): 3414, 2918, 2854, 1649, 1451, 1096; ¹H-NMR (CDCl₃, 500 MHz); δ 7.36-7.25 (m, 30H); 6.16 (d, *J* = 8, 1H); 4.86-4.56 ( m, 11H); 4.43 (d, *J* = 12, 1H); 4.23 (m, 1H); 4.01 (m, 1H); 3.98 (dd, *J* = 9, 4, 1H); 3.82 (m, 3H); 3.73 (t, *J* = 4, 1H); 3,57 (dd, *J* = 11, 5, 1H); 3.17 (t, *J* = 4, 1H); 2.95 (dd, *J* = 11,4, 1H); 2.62 (dd, *J* =11, 5, 1H); 1.98 (m, 2H), 1.66 (m, 2H); 1.54 (m, 2H); 1.28 (m, 32H); 0.91 (t, *J* = 7, 3H); 0.85 (t, *J* = 7, 3H). ¹³C-NMR (CDCl₃, 100 MHz): δ 172.3, 138.8, 138.7, 138.3(2C), 138.2, 137.9, 128.4-127.4 (30C), 82.4, 81.8, 81.5, 80.4, 79.1, 79.0, 75.9, 75.6, 73.8, 73.1, 72.4, 71.4, 67.3, 58.2, 48.6, 48.5, 36.7, 31.8, 30.2, 29.7-29.3 (13C), 25.6, 25.4, 22.6, 14.1, 14.0, HRMS. Calculated for C₇₄H₁₀₀N₂O₈ (M+H⁺): 1145.7558. Found: 1145.7560.

### Debenzylation reactions of products derived from reductive amination

### Example 9:

### (2S,3S,4R)-2-octanamido-1-(1'rs,2'RS,3'SR,4'SR,5'RS,6'SR)-2',3',4',5',6'-pentahydroxycyclohexylamino)octadecane-3,4-diol (Compound 1a).

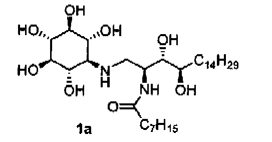

A solution in dichloromethane (2 mL) of 53 mg (0.05 mmol) of (2*S*,3*S*,4*R*)-(3,4-dibenzyloxy-1-(1'*rs*,2'*RS*,3'*SR*,4'*SR*,5'*RS*,6'*SR*)-2',3',4',5',6'-pentabenxyloxycyclohexylamino)aatadecan-2-yl)actanamide is cooled to -78 °C by an external bath and is maintained under argon. A solution of BCl₃ (1M) in heptane (2 equiv- for each OBn group) is added. The reaction mixture is allowed to reach room temperature and is stirred for 16 h. After this time, it is cooled again at -78 °C, and 2 mL of methanol are added dropwise. The bath is removed and it is allowed to reach room temperature, after what it is concentrated *in vacuo.* Then, AcOEt (3 mL) is added to the resultant residue and is introduced in an ultrasound bath for 5-7 minutes. The resultant solid is collected by filtration. It is washed with AcOEt (3x1 mL) and dried *in vacuo,* to give 27 mg (0.042 mmol, 85%) of the compound in the form of an hydrochloride.

Pf = 241-243. [α]_{D} = +9.6 (MeOH, c, 0.8); lR (film): 3358, 2931, 2852, 1657, 1458, 1111. ¹H-NMR (CD₃OD, 500 MHz); δ4.37(q, *J* = 5,5, 1H): 3.68 (t, *J* = 5.5, 1H); 3.63 (dd, *J* = 12, 5.5, 1H); 3.55 (m, 3H); 3.39 (dd, *J* = 13, 6, 1H); 3.33-3.2 (m, 4H); 3.05 (t, *J* = 11, 1H), 2.29 (t, *J* = 7, 2H); 1,64 (m, 2H), 1.29 (m, 34H); 0.90 (t, *J* = 7, 6H); ¹³C-NMR (CD₃OD, 100 MHz): δ 177.6, 77.9, 77.4 (2C), 76.0, 74.1, 71.2, 71.1, 64.5, 49.6, 49.4, 37.9. 33.9. 33.6; 31.7-31.3 (10 C), 31.3, 31.2, 31.0, 27.8, 27.6, 24.6, 15.3 (2C). HRMS. Calculated for C₃₂H₆₄N₂O₈ (M+H+): 605,4741. Found: 605.4739.

### Example 10:

### (2S,3S,4R)-2-Hexacosanamide-1-(1'rs,2'RS,3'SR,4'sr,5'RS,6'SR)-2',3',4',5',6'-pentahydroxycyclohexylamino)octadecane-3,4-diol (Compound 1b).

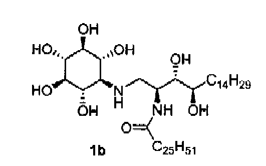

It is prepared by debenzylation with BCl₃ according to the procedure of example 9 of the product (2*S*,3*S*,4*R*)-(3,4-dibenzyloxy-1-(1'*rs*,2'*Rs*,3'*SR*,4'*SR*,5'*RS*,6'*SR*)-2',3',4',5',6'-pentabenzyloxycyclohexylaminooctadecan-2-yl)hexacosanamide. 83% yield.

Pf = 242-244. [α]_{D} = +8.1 (MeOH, c, 0.8). IR (film): 3363 (br), 2922, 2845, 1649, 1455, 1069. ¹H-NMR (CD₃OD, 400 MHz); δ 4.36 (m, 1H); 3.65 (dd, *J* = 6, 4.8, 1H); 3.56 (m, 2H); 3.48 (m, 2H); 3.36-3.13 (m, 5H); 3.01 (t, *J* = 10, 1H), 2.26 (t, *J* = 7, 2H); 1.61 (m, 2H), 1.29 (m, 70H); 0.90 (t, *J* = 7, 6H); ¹³C-NMR (DMSO, 60 °C, 100 MHz): δ 173.9, 78.1, 75,6 (2C), 74.5, 71.6, 69.7, 69.6, 63.3, 48.3, 48.1, 36.2, 32.7, 31.9; 29.7-29.3 (31 C). 26.0. 25.7, 22.7 14.4 (2C). HRMS. Calculated for C₅₀H₁₀₀N₂O₈ (M+H⁺); 857.7558. Found: 857,7563.

### Example 11:

### (2S,3S,4R)-octanamido-1-(1'R,2'S,3'R,4'S,5'S,6'S)-2',3',4',5',6'-pentahydroxycyclohexylamino)octadecane-3,4-diol (Compound 3b)

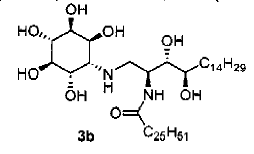

It is prepared by debenxylation with BCl₃ according to the procedure of example 9 of the product (2*S*,3*S*,4*R*)-(3,4-dibenzyloxy-1-(1'*R*,2'*S*,3'*R*,4'*S*,5'*S*,6'*S*)-2',3',4',5',6'-pentabenzyloxycyclohoxylemino)ootadecan-2-yl)octanamide. 84% yield.

Pf = 214-216. [α]_{D} = -6.8 (MeOH, c, 0.75); IR (film): 3417, 2931, 2850, 1650,1443,1034, ¹H-NMR (CD₃OD, 400 MHz); δ 4.36 (q, *J* = 5.2, 1H); 4.15 (m, 2H), 3.90 (m, 2H); 3.86 (m, 1H); 3.65 (t, *J* = 5, 1H); 3.57 (m, 2H), 3.40 (dd, *J* = 12.5, 6, 1H); 3.20 (dd, *J* = 12.5, 6, 1H), 2.23 (t, *J* = 7, 2H); 1,60 (m, 2H), 1.29 (m, 34H); 0.88 (t, *J* = 7, 6H), ¹³C-NMR (CD₃OD, 100 MHz): δ 176.7, 77.0, 74.7, 73.6, 72.9. 72.8, 69.9, 66.3, 59.9, 48.4, 47.8, 36.9, 33.5, 33.0, 32.8, 30.7 (8C), 30.4, 30.3, 30.1, 26.9, 26.7, 23.7 (2C), 14.4 (2C). HRMS. Calculated for C₃₂H₆₄N₂O₈ (M+H⁺): 605.4741. Found: 605.4738.

### Example 12:

### (2S,3S,4R)-2-hexacosanamide-1-(1'R,2'S,3'R,4'S,5'S,6'S)-2',3',4',5',6'-pentahydroxycyclohexylamino)octadecane-3,4-diol (Compound 3a).

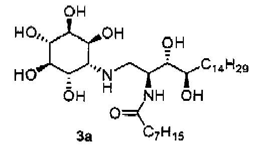

It is prepared by debenzylation with BCI₃ according to the procedure of example 9 of the product (2*S*,3*S*,4*R*)-(3,4-dibenzyloxy-1-1'*R*,2'*S*,3'*R*,4'*S*,5'*S*,6'*S*)-2',3',4',5',6'-pentabenzyloxycyclohexylaminooctadecan-2-yl)hexacosanamide. 89% yield.

Pf = 235-237: [α]_{D} = -9.3 (MeOH, c, 0.75). IR (film): 3368, 2923, 2847, 1645, 1461, 1042. ¹H-NMR (CD₃OD, 45 °C, 400 MHz); δ 4.37(q, *J* = 5, 1H); 4.16 (m, 2H), 3.98 (m, 2H); 3,88 (m, 1H); 3.67 (t, *J* = 5, 1H); 3.57 (m, 2H), 3.40 (dd, *J* = 10_{,} 5.2, 1H); 3,21 (dd, *J* = 10. 5.2, 1H), 2.23 (t, *J* = 7, 2H); 1.60 (m, 2H), 1.29 (m, 70H); 0.88 (t, *J* = 7, 6H). ¹³C-NMR (CD₃OD, 45 °C, 100 MHz): δ 176.7, 77.0, 74.7, 73.6, 72.9. 72.8, 69.9, 66.3, 59.9, 48.4, 47.8, 36.9, 33.5, 33.0, 30,7-30.4 (31C), 26.9, 26.8, 23.7, 14.4 (2C). HRMS. Calculated for C₅₀H₁₀₁N₂O₈ (M+H⁺): 857.7558, Found: 857.7580.

### Preparation of tetrahydroxycyclohexylamines

### Example 13:

### (1R,2S,3R,4R,5S)-2,3,4,5-tetrakis(benzyloxy)cyclohexanol

A solution of O-tetrabenzylconduritol B [González-Bulnes et al Carbohydr Res 2007, 342, 1947-52] (1 g, 1.97 mmol) in THF (50 mL) is stirred at 0 °C while BH₃.THF (1 M in THF, 6 mL, 6 mmol) is added dropwise for about 5 min. The reaction mixture is brought to room temperature and the stirring is continued for 16 h. NaOH (1 M aq., 8 mL), and hydrogen peroxide (30% w/v aq., 4 mL) are added and stirred vigorously for 1 h at room temperature. It is extracted with ether and the combined organic phases are washed with water and a saturated solution of NaCl, dried (Na₂SO₄) and concentrated *in vacuo* to give a solid that is purified by flash chromatography (Hexane:AcOEt, 7:3), to give a white solid (723 mg, 70%).

[α]_{D} = +8 (CHCl₃, C, 1); ¹H-NMR (CDCl₃, 500 MHz); δ 7,34 (m, 20H); 5.03-4.95 (m, 3H); 4.87 (t, *J* = 10, 2H); 4.89 (m, 3H); 3.61 (t, *J* = 9, 1H); 3.53 (m, 3H); 3.36 (t, *J* = 9, 1H); 2.37 (dt, *J* = 13, 4, 1H); 2.33 (br, 1H); 1.48 (q, *J* = 13, 1H), ¹³C-NMR (CDCl₃, 75 MHz): δ 138.6, 138.4 (2C), 138.2, 128.6-127.6 (20C), 85.8 (2C), 83.3, 77.4, 75.8 (2C), 75.4, 72.3, 68.3, 33.9, HRMS. Calculated for C₃₄H₃₆O₅ (M+H⁺): 525.2641, Found: 525.2644.

### Example 14:

### (1S,2S,3R,4R,5S)-2,3,4,5-tetrakis(benzyloxy)cyclohexanol

A solution of (1*R*,2*S*,3*R*,4*R*,5*S*)-2,3,4,6-tetrakis(benzyloxy)cyclohexanol (540 mg, 1.02 mmol) prepared according to example 9 and triethylamine (0.44 mL. 3 mmol) in THF (15 mL) is treated with MsCl (methanesulfonyl chloride) (232 mg, 2.04 mmol), It is stirred at room temperature for 3 h, diluted with H₂O (20 mL), and extracted with AcOEt (3x15 mL). The extracts are dried with Na₂SO₄, filtered and evaporated to give the intermediate mesylate, that is dissolved in DMF (dimethylformamide)/H₂O (98/2) (10 mL) and stirred at 140°C in a closed tube for 96 h. The solvent is removed at low pressure and the resultant residue is treated with Et₂O (10 mL), washed with H₂O (3X10 mL), dried and evaporated to give an oil that is purified by flash chromatography (hexane/AcOEt 7:3) to give the alcohol (60 mg, 30%).

Pf = 65-67; [α]_{D} = -4 (CHCl₃, C, 1); IR (neat, cm⁻¹): 3361, 2943, 1427, 1118,986. ¹H-NMR (CDCl₃, 400 MHz); δ 7.33 (m, 20H); 4.95-4.65 (m, 8H); 4.12 (m, 1H); 3.95 (m, 1H); 3.84 (t, *J* = 9.2, 1H); 3.49 (m, 2H); 2.84 (dt, *J* = 14, 4, 1H); 1.40 (ddd, *J* = 14, 12, 2.4, 1H), ¹³C-NMR (CDCl₃, 75 MHz): δ 138.8, 138.7, 138.6, 137.9, 128,5-127.4 (20 C), 85.7, 82.8, 81.5, 77.0, 76.0, 75.7, 72.9, 72.8, 65.9, 32.4. HRMS. Calculated for C₃₄H₃₈O₅ (M+H⁺): 525.2641. Found: 525.2644.

### Example 15:

### (1S,2S,3R,4R,5S)-2,3,4,5-tetrakis(benzyloxy)cyclohexanamine

A solution of (1*R*,2*S*,3*R*,4*R*,5*S*)-2,3,4,5-tetrakis(benzyloxy)cyclohexanol (mg, 0.28 mmol) prepared according to example 9 and triethylamine (0.12 mL, 0.84 mmol) in dichloromethane (10 mL) is treated with MsCl (43 µL, 0.56 mmol) at 0 °C. The mixture is stirred and brought to room temperature for two hours. Dichloromethane (10 mL) is added and it is washed three times with NaOH 1N (10 mL). The organic phase is washed with water and dried with anhydrous Na₂SO₄, it is filtered and concentrated *in vacuo* to give an oil that is dissolved in DMF (5 mL). NaN₃ (182 mg, 2.8 mmol) is added and the mixture is heated at 90 °C for 12 hours. The mixture is cooled at room temperature, diethyl ether (20 mL) is added, and it is extracted with a NaCl saturated aqueous solution (3x20 mL). The organic phase is dried over Na₂SO₄, filtered and concentrated *in vacuo* to give the intermediate azide that is purified by flash chromatography in silica gel (hexane/AcOEt 9:1). (109 mg, 71%), white solid: Pf = 87-89, [α]_{D} = -8.5 (CHCl₃, C, 1).

The azide is reduced to amine by LiAlH₄ (13 mg, 0.36 mmol) that is added in a portion to a solution thereof in THF (10 mL) at 0 °C and stirred at this temperature 1 h, and for 1 h at room temperature. After cooling at 0 °C the reaction mixture, 0.3 mL of a Na₂SO₄ saturated aqueous solution are added dropwise. The salts are filtered over Celite® that is washed with Et₂O, and the filtrates are evaporated *in vacuo* to give the pure amine in the form of a white solid (96 mg, 93%).

Pf = 74,76. [α]_{D} = +2 (CHCl₃, C, 1); IR (film , cm⁻¹): 3351, 2957, 2362, 1433, 1112. ¹H-NMR (CDCl₃, 400 MHz); δ 7.30 (m, 20H); 4.87 (m, 4H); 4.69 (m, 4H); 3.96 (m, 2H); 3.46 (m, 3H); 2.80 (dt, *J* = 14, 3.2, 1H); 1.43 (m, 1H). ¹³C-NMR (CDCl₃, 100 MHz); δ: 139.0, 138.9, 138.7, 138.5, 128.4-127.4 (20C), 86.2, 83.1, 81.4, 77.1, 75.9, 75.6, 72.8, 72.4, 46.8, 33.5. HRMS. Calculated for C₃₄H₃₇NO₄ (M+H+): 524.2801. Found: 524.2821.

### Example 16:

### (1R,2S,3R,4R,5S)-2,3,4,5-tetrakis(benzyloxy)cyclohexanamine

It is prepared from (1*S*,2*S*,3*R*,4*R*,5*S*)-2,3,4,5-tetrakis(benzyloxy)cyclohexanol according to the procedure described in example 11 to give a white solid (45 mg, 55%).

Pf = 102-103. [α]_{D} = -11.5 (CDCl₃, C, 1.0). IR (neat, cm⁻¹): 3355, 2923, 2366, 1454, 1069. ¹H-NMR (CDCl₃, 400 MHz); δ 7.31 (m, 20H); 4.98 (m, 3H); 4.84 (m, 2H); 4,67 (m, 3H), 3.54 (m, 3H); 3.18 (t, *J* = 9.2, 1H); 2.71 (m, 1H); 2.20 (dt, *J* = 13.2, 4, 1H); 1.57 (br, 2H, NH2), 1.31 (m, 1H). 13C-NMR (CDCl₃, 100 MHz): δ 138.6, 138.4 (2C), 138.3, 128.5-127.5 (20C), 86.8, 86.0, 84.4, 78.4, 75.7 (2C), 75.5, 72.3, 49.8, 35.1. HRMS. Calculated for C₃₄H₃₇NO₄ (M+H⁺): 524.2801. Found: 524.2819.

### Substitution reactions of phytosphingosine-derived aziridines

### Example 17:

### Preparation of N-((1S)-1-((4S,5R)-2,2-dimethyl-5-tetradecyl-1,3-dioxalan-4-yl)-2-((1S,2S,3R,4R,5S)-2,3,4,5-tetrakis(benzyloxy)cyclohexylamino)ethyl)-2-nitrobenzenesulfonamide

To a solution of 70 mg (0.13 mmol) of (1*S*,2*S*,3*R*,4*R*,5*S*)-2,3,4,5-tetrakis(benzyloxy)cyclohexanamine in dried MeCN (5 mL), 68 mg (0.13 mmol) of (*S*)-2-((4*S*,5*R*)-2,2-dimethyl-5-tetradecyl-1,3-dioxolan-4-yl)-1-(2-nitrophenylsulfonyl)aziridine (cfr. Y. Harrak et al. Eur J Org Chem 2008, 4647-4654) are added. The mixture is stirred at reflux for 3h, is cooled at room temperature and concentrated *in vacuo,* The residue is purified by column chromatography (hexane/AcOEt 4:1) to give a colorless oil (118 mg, 87%).

[α]_{D} = +5.1 (CDCl₃, C, 1.0). IR (film, cm⁻¹): 3316, 2924, 2853, 1541, 1306, 1070, 697. ¹H-NMR (CDCl₃, 500 MHz); δ 8.12 (d, *J* = 7.5, 1H); 7.65 (t, *J* = 7, 1H); 7.56 (m, 2H); 7.31 (m, 20H); 4.84 (m, 4H); 4.60 (m, 4H); 4,13 (m, 2H); 3.85 (m, 3H); 3.53 (dd, *J* = 9.5, 4, 1H); 3.49 (t, *J* = 9, 1H); 3.01 (d, *J* = 2.5, 1H); 2.87 (dd, *J* = 12, 4, 1H); 2.72 (dd, *J* = 12, 3.5, 1H); 2.17 (dt, *J* = 14, 3.5, 1H); 1.51 (m, 3H); 1.40 (s, 3H); 1.27 (m, 27 H); 0.91 (t, *J* = 7, 3H). ¹³C-NMR (CDCl₃, 100 MHz): δ 147.7, 139.1, 139.0, 138.9, 138.5, 135.3, 133.3, 132.7, 129.7, 128.3-127.3 (20C), 125.3, 107.9, 85.7, 82.6, 81.6, 78.2, 77.5, 77.4, 75.8, 75.4, 72.7, 72.5, 54.1, 53.4, 49.1, 31.9, 29.8-29.3 (11C), 27.4, 26.7, 25.2, 22.7, 14.1. HRMS. Calculated for C₆₁H₈₁N₃O₁₀S (M+H⁺): 1048.5721. Found: 1048.5742.

### Example 18:

### /V-((S)-1-((4S,5R)-2,2-dimethyl-5-tetradecyl-1,3-dioxolan-4-yl)-2-((1R,2S,3R,4R,5S)-2,3,4,5-tetrakis(benzyloxy)cyclohexylamino)ethyl)-2-nitrobenzenesulfonamide

According to the procedure of example 17, (1*R*,2*S*,3*R*,4*R*,5*S*)-2,3,4,5-tetrakis(benzyloxy)cyclohexanamine and (*S*)-2-((4*S*,5*R*)-2,2-dimethyl-5-tetradecyl-1 ,3-dioxolan-4-yl)-1-(2-nitrophenylsulfonyl)aziridine (cfr. Y. Harrak et al., Eur J Org. Chem 2008, 4647-4654) are reacted to give a colorless oil (71 mg, 87%).

[α]_{D} = +30 (CDCl₃, C, 1.0); IR (film): 3340, 2924, 2853, 1593, 1365, 1072. ¹H-NMR (CDCl₃, 400 MHz); δ 8.03 (d, *J* = 7.6, 1H); 7.61 (t, *J* = 7.6, 1H); 7.52 (t, *J* = 7.6, 1H); 7.44 (d, *J* = 8, 1H), 7.28 (m, 21H); 4.96-4.54 (m, 8H); 4.08 (m, 2H); 3.66 (m, 1H), 3.44 (m, 3H); 3.16 (t, *J* = 9.2, 1H); 2.80 (d, *J* = 10, 1H); 2.55 (d, *J* = 10, 1H); 2.42 (t, *J* = 10, 1H); 2.23 (m, 1H); 1.51 (m, 3H); 1.33 (s, 3H); 130 (s, 3H), 1.27 (m, 24H); 0.87 (t, *J* = 7, 3H); ¹³C-NMR (CDCl₃, 100 MHz): δ 147.7, 138.6, 138.4 (2C), 138.3, 135.3, 133.3, 132.6, 130.0, 128.4-127.4 (20 C), 125.1, 107.8, 85.8, 84.8, 82.9, 78.3, 77.5, 76.6, 75.7 (2C), 74.8, 72.3, 55.4, 54.3, 45.7, 32.0, 31.8, 29.6-29.2 (10 C), 27.8, 26.3, 25.5, 22.6, 14.1, HRMS. Calculated for C₆₁H₈₁N₃O₁₀S (M+H+): 1048,5721. Found: 1048.5739.

### Deprotection of 2-nitrobenzenesulfonamides

### Example 19:

### (S)-1-((4S,5R)-2,2-dimethyl-5-tetradecyl-1,3-dioxolan-4-yl)-N²-

### ((1S,2S,3R,4R,5S)-2,3,4,5-tetrakis(benzyloxy)cyclohexyl)ethane-1,2-diamine

To a solution of *N*-((1*S*)-1-((4*S*,5*R*)-2,2-dimethyl-5-tetradecyl-1,3-dioxolan-4-yl)-2-((1*S*,2*S*,3*R*,4*R*,5*S*)-2,3,4,5-tetrakis(benzyloxy)cyclohexylamino)ethyl)-2-nitrobenzenesulfonamide (105 mg, 0.1 mmol) in dried CH₃CN (2 mL), thiophenol (44 mg, 0.4 mmol) and Cs₂CO₃ (98 mg, 0.3 mmol) are added. It is stirred at room temperature for 24 hours and a NaHCO₃ saturated aqueous solution (20 mL) is added, the aqueous phase being extracted with CH₂Cl₂. The combined organic extracts are dried over MgSO₄, filtered and concentrated at low pressure to give an oil that is purified by column chromatography (CH₂Cl₂/MeOH 99:1) to give a clear liquid (73 mg, 85%).

[α]_{D} = +10.0 (CDCl₃, C, 1.0); IR (film): 3350, 2920, 2832, 1449, 1367, 1081. ¹H-NMR (CDCl₃, 500 MHz); δ 7.31 (m, 20H); 4.87 (m, 4H); 4.67 (m, 4H); 4.12 (m, 1H); 3.91 (m, 1H); 3.75 (dd, *J* = 9.5, 5.5, 1H); 3.55 (dd, *J* = 9.5, 4, 1H); 3.48 (m, 2H); 3.11 (m, 1H); 2.88 (m, 1H); 2.69 (dd, *J* = 12, 3, 1H), 2.56 (dd, *J* = 12, 6.5, 1H); 2.23 (dt, *J* = 13.5, 4.5, 1H); 1.54 (m, 3H); 1.42 (s, 3H); 1.30 (s, 3H); 1.27 (m, 24H); 0.90 (t, *J* = 7, 3H). ¹³C-NMR (CDCl₃, 100 MHz): δ 138.9 (2C), 138.7, 138.4, 128.3-127.4 (20C), 107.7, 86.2, 83.1, 82.1, 80.0, 77.9, 76.7, 75.9, 75.7, 72.7, 72.2, 53.6, 51.8, 50.2, 31.9, 30.2-29.3 (11C), 28.3, 26.2, 25.9, 22.6, 14.1. HRMS. Calculated for C₅₅H₇₈N₂O₆ (M+H⁺⁾: 863.5938. Found: 863.5942.

### Example 20:

### (S)-1-((4S,5R)-2,2-dimethyl-5-tetradecyl-1,3-dioxolan-4-yl)-N²-((1R,2S,3R,4R,5S)-2,3,4,5-tetrakis(benzyloxy)cyclohexyl)ethane-1,2-diamine

According to the procedure described in example 19, (*S*)-1-((4*S*,5*R*)-2,2-dimethyl-5-tetradecyl-1,3-dioxolan-4-yl)-*N*²-((1*R*,2*S*,3*R*,4*R*,5*S*)-2,3,4,5-tetrakis(benzyloxy)cyclohexyl)ethane-1,2-diamine is reacted with thiophenol and cesium carbonate to give the product in the form of an oil with an 82% yield.

[α]_{D} = -7 (CDCl₃, C, 1.0); R (film): 3402, 2924, 2853, 1454, 1367, 1069. ¹H-NMR (CDCl₃, 400 MHz); δ 7.33 (m, 20H); 5.02-4.68 (m, 8H); 4.13 (m, 1H); 3.77 (dd, *J* = 8.4, 5.6, 1H); 3.56(m, 3H); 3.38 (t, *J* = 8.8, 1H); 2.84 (dd, *J* = 11.2, 3.2, 1H); 2.77 (dt, *J* = 8,4, 2.8, 1H), 2.52 (m, 1H); 2,35 (dt, *J* = 12.8, 4, 1H); 1.50 (m, 3H): 1.39 (s. 3H); 1.31 (s, 3H): 1.28 (m, 24H): 0.90 (t, *J* = 7.2, 3H). ¹³C-NMR (CDCl₃, 100 MHz): δ 138.7, 138.5, 138.4, 138.3, 128.4-127.5 (20C), 107.8, 85,8, 84.7, 84.2, 80.6, 78.5, 77.8, 75.8, 75.7, 75.4, 72.4, 55.6, 50.8, 50.1, 32.4, 31.9, 30.2-29.3 (10C), 28.3, 26.0, 25.9, 22.6, 14.1. HRMS. Calculated for C₅₅H₇₈N₂O₆ (M+H⁺): 863.5938. Found: 863.5943.

### Amines acylation reactions

### Example 21:

### (2S,3S,4R)-(3,4-isopropylidenedioxy-1-(1'S,2'S,3'R,4'S,5'S)-2',3',4',5'-tetrabenzyloxycyclohexylaminooctadecan-2-yl)hexacosanamide

To a solution of (*S*)-1-((4*S*,5*R*)-2,2-dimethyl-5-tetradecyl-1,3-dioxolan-4-yl)-*N*²-((1*S*,2*S*,3*R*,4*R*,5*S*)-2,3,4,5-tetrakis(benzyloxy)cyclohexyl)ethane-1,2-diamine (70 mg, 0.08 mmol) and cerotic acid (32 mg, 0.08 mmol) in dried THF (10 mL), EDC (23 mg, 0.12 mmol) is added. The reaction is heated to reflux for 12 h, cooled at room temperature, and water (10 mL) is added, It is extracted with AcOEt, washed with water, sat. NaCl, and the organic phase is dried with MgSO₄. It is filtrated and concentrated *in vacuo* to give an oil that is purified by column chromatography in silica gel (hexane/AcOEt 7/3) to give a white solid (67 mg, 68%).

Pf = 72-74. [α]_{D} = +16.2 (CDCl₃, C, 1.0); IR (film, cm⁻¹): 3325, 2918, 2850, 1650, 1470, 1095. ¹H-NMR (CDCl₃, 500 MHz); δ 7.32 (m, 20H); 5.98 (d, *J* = 9.5, 1H); 4.93 (d, *J* = 11, 1H); 4.87 (m, 2H); 4.83 (d, *J* = 11, 1H); 4.65 (m, 3H); 4.58 (d, *J* = 11, 1H); 4.19 (m, 1H); 4.13 (m, 1H); 4.00 (t, *J* = 6,5, 1H): 3.93 (t, *J* = 9.5, 1H); 3.87 (m, 1H); 3.09 (d, *J* = 3, 1H); 2.80 (dd, *J* = 12, 3.5, 1H); 2.20 (dt, *J* = 15.5, 4, 1H); 2.10 (m, 2H); 1.56 (m, 3H), 1.40 (s, 3H); 1.27 (m, 73H); 0.89 (t, *J* = 7, 6H). ¹³C-NMR (CDCl₃, 100 MHz): δ 172.3, 138.8 (2C), 138.4, 138.3, 128.4-127.5 20C), 107.7, 85.8, 82.8, 82.0, 77.9, 77.8, 77.7, 75.9, 75.8, 72.9, 72.4, 53.2, 48.5, 47.7, 37.0, 31.9, 29.7-29.3 (33C), 27.7, 26.6, 25.8, 25.3, 22.7, 14.1(2C). HRMS. Calculated for C₈₁H₁₂₈N₂O₇ (M+H⁺): 1241.9800. Found: 1241.9766.

### Example 22:

### (2S,3S,4R)-(3,4-isopropylidenedioxy-1-(1'R,2'S,3'R,4'S,5'S)-2',3',4',5'-tetrabenzyloxycyclohexylaminooctadecan-2-yl)hexacosanamide

By reaction of (*S*)-1-((4*S*,5*R*)-2,2-dimethyl-5-tetradecyl-1,3-dioxolan-4-yl)-*N*²-((1R,2S,3R,4R,5S)-2,3,4,5-tetrakis(benzyloxy)cyclohexyl)ethane-1,2-diamine with cerotic acid and EDC, according to the procedure of example 21, a white solid is obtained with a 63% yield.

Pf = 85-87 °C. [α]_{D} = -8 (CHCl₃, C, 1.0); IR (neat, cm-1): 3331, 2925, 2849, 1664, 1444, 1111. ¹H-NMR (CDCl₃, 500 MHz); δ 7.34 (m, 20H); 5.97 (br, 1H); 5.04-4.68 (m, 8H); 4.02 (m, 2H); 3.95 (m, 1H); 3.52 (m, 3H); 3.31 (m, 1H); 3.02 (d, *J* = 12.5, 1H); 2.64 (d, *J* = 13, 1H); 2.46 (m, 1H); 2.29 (m, 1H); 1.96 (m, 2H), 1.62 (br, 1H), 1.55 (m, 3H), 1.40 (s, 3H); 1.32 (s, 3H), 1.26 (m, 70H); 0.89 (t, *J* = 7, 6H). ¹³C-NMR (CDCl₃, 100 MHz): δ 172.5, 138.6, 138.4 (2C), 138.3, 128.5-127.6 (20C), 107.7, 85.8, 84.7, 84.6, 78.5, 77.7, 77.0, 75.9, 75.8, 75.7, 72.6, 57.1, 48.3, 46.9, 36.7, 33.4, 31.9, 29.7-29.3 (31 C), 29.0, 27.9, 26.5, 25.7, 25.3, 22.7, 14.1 (2C). HRMS. Calculated for C₈₁H₁₂₈N₂O₇ (M+H⁺): 1241.9800. Found: 1241.9773.

### Debenzylation reactions of products derived from aziridine openings

### Example 23:

### (2S,3S,4R)-2-hexacosanamide-1-(1'S,2'S,3'R,4'R,5'S)-2,3,4,5,-tetrahydroxycyclohexylaminooctadecane-3,4-diol (Compound 4b).

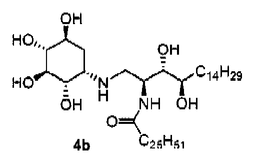

According to the procedure described in example 9, 30 mg (0,024 mmol) of (2*S*,3*S*,4*R*)-(3,4-isopropylidenedioxy-1-(1'*S*,2'*S*,3'*R*,4'*R*,5'*S*)-2',3',4',5'-tetrabenzyloxycyclohexylaminooctadecan-2-yl)hexacosanamide are treated with BCl₃ (1M in heptane, 0.24 mL, 0.24 mmol), To give 15 mg (0.015 mmol, 73%) of the (2*S*,3*S*,4*R*)-2-hexacosanamide-1-(1'*S*,2'*S*,3'*R*,4'*R*,5'*S*)-2,3,4,5,-tetrahydroxycyclohexylaminooctadecane-3,4-diol hydrochloride in the form of a white solid.

Pf = 213-215. [α]_{D} = -25 (MeOH, C, 0.2) ; IR (neat, cm⁻¹): 3351 (br), 2942,2833,1642,1433,1021; ¹H-NMR (MeOH, 500 MHz, 50°C); δ 4.33 (c, *J* = 5, 1H); 3.90 (dd, *J* = 7, 4, 1H); 3.84 (m, 1H); 3.65 (m, 4H); 3.53 (m, 2H); 3.13 (dd, *J* = 13, 6.5, 1H); 2.27 (m, 2H); 1.78 (m, 1H); 1.64 (m, 3H); 1.32 (m, 70H); 0.92 (t, *J* = 7, 6H). ¹³C-NMR (DMSO, 100 MHz): δ 174.0, 75.9, 75.5, 72.8, 71.8, 70.5, 68.2, 56.6, 48.2, 46.5, 36.1,33.1, 31.9, 29.7-29.3 (31 C), 28.8, 25.9, 25.8, 22.7, 14.5 (2C). HRMS. Calculated for C₅₀H₁₂₇N₂O₇ (M+H⁺): 841.7609. Found: 841.7628.

### Example 24:

### (2S,3S,4R)-2-hexacosanamide-1-(1'R,2'S,3'R,4'R,5'S)-2,3,4,5,-tetrahydroxycyclohexylaminooctadecane-3,4-diol (Compound 2b).

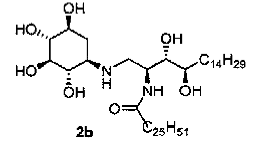

According to the procedure described in example 9, 30 mg (0.024 mmol) of (2*S*,3*S*,4*R*)-(3,4-isopropylidenedioxy-1-(1'*S*,2'*S*,3'*R*,4'*R*,5'*S*)-2',3',4',5'-tetrabenzyloxycyclohexylaminooctadecan-2-yl)hexacosanamide are treated with BCl₃ (1M in heptane, 0.24 mL, 0.24 mmol). 10 mg (0.011 mmol, 46%) of the (2*S*,3*S*,4*R*)-2-hexacosanamide-1-(1'*R*,2'*S*,3'*R*,4'*R*,5'*S*)-2,3,4,5-tetrahydroxycyclohexylaminooctadecane-3,4-diol hydrochloride are obtained in the form of a white solid,

Pf = 227-229. [α]_{D} = +12 (MeOH, C, 0.2); IR (neat, cm⁻¹): 3343 (br), 29512, 2812, 1668, 1427, 1007; ¹H-NMR (DMSO, 500 MHz, 60°C); 4.22 (m, 1H); 3.81 (m, 1H); 3.48-2.98 (m, 8H); 2.13 (m, 3H); 1.46 (m, 5H); 1.26 (m, 68H); 0.87 (m, 6H). HRMS. Calculated for C₅₀H₁₂₇N₂O₇ (M+H⁺): 841.7609. Found: 841.7628.

### Preparation of the compounds of formula (I) from other aminocyclitols

### Example 25:

### (2S,3S,4R)-2-hexacosanamide-1-(1'S,2'S,3'R,4'R,5'S,6'S)-2,3,4,5-tetrahydroxy-6-methoxycyclohexylaminooctadecane-3,4-diol

This compound was prepared in four steps: 1) following the method described in example 17 by reaction of (1*S*,2*S*,3*R*,4*R*,5*S*,6*S*)-2,3,4,5-tetrakis(benzyloxy)-6-methoxycyclohexanamine with *(S*)-2-((4*S*,5*R*)-2,2-dimethyl-5-tetradecyl-1,3-dioxolan-4-yl)-1-(2-nitrophenylsulfonyl)aziridine [Y. Harrak et al. Eur J Org. Chem 2008, 4647-4654] to give the corresponding amine (79% yield); 2) deprotection of the nitrobenzenesulfonyl group according to the procedure described in example 19 to give de compound (*S*)-1-((4*S*,5*R*)-2,2-dimethyl-5-tetradecyl-1,3-dioxolan-4-yl)-*N*²-((1'*S*,2'*S*,3'*R*,4'*R*,5'*S*,6'*S*)-2,3,4,5-tetrakis(benzyloxy)-6-methoxycyclohexyl)ethane-1,2-diamine (78% yield); 3) acylation with cerotic acid of the primary amine following the procedure described in example 21 to give the amide with a 76% yield; and 4) deprotection of the hydroxylated substituents by reaction with BCl₃ following the procedure detailed in example 9 to give with a 91% yield the (2*S*,3*S*,4*R*)-2-hexacosanamide-1-(1'*S*,2'*S*,3'*R*,4'*R*,5'*S*,6'*S*)-2,3,4,5,-tetrahydroxy-6-methoxycyclohexylamino)octadecane-3,4-diol hydrochloride in the form of a dense oil.

[α]_{D} = -9 (MeOH, 0.5); IR (film, cm⁻¹): 3364 (br), 2948, 2828, 1871, 1436, 1063. 1H-NMR (CD3OD, 500 MHz, 50°C); δ 4.34 (m, 1H); 4.26 (m, 1H), 4.20 (m, 1H); 4.04 (m, 1H); 3.91 (m, 1H); 3.86 (m, 1H); 3.70 (m, 2H); 3.47 (s, 3H); 3.25 (m, 1H); 2.25 (m, 2H); 1.60 (m, 4H); 1.28 (m, 68H); 0.90 (m, 6H). 13C-NMR (CD3OD, 100 MHz, 55°C): δ 177.5, 77.8, 76.8, 74.6, 73.8, 73.6, 71.0, 59.5, 58.0, 50.1, 49.6, 48.8, 37.9, 34.5, 33.8 (2C), 31.6-31.2 (29C), 27.8, 27.6, 24.5 (2C), 14.2(2C). HRMS. Calculated for C₅₁H₁₀₃N₂O₈ (M+H⁺): 871.7714. Found: 871.7755.

### Example 26:

### (2S,3S,4R)-2-Hexacosanamide-1-(1'S,4'S,5'S,6'S)-4',5',6'-trihydroxycyclohexenylaminooctadecane-3,4-diol

This compound was prepared in four steps: 1) following the method described in example 17 by reaction of (1*S*,4*S*,5*S*,6*S*)-4,5-isopropylidenedioxy-3-hydroxycyclohexenylamine with *(S*)-2-((4*S*,5*R*)-2,2-dimethyl-5-tetradecyl-1,3-diaxalan-4-yl)-1-(2-nitrophenylsulfonyl)aziridine [Y. Harrak et al. Eur J Org. Chem 2008, 4647-4654] to give the corresponding amine (97 % yield); 2) deprotection of the 2-nitrobenzenesulfonyl group according to the procedure described in example 19 to give the compound (*S*)-1-((4*S*,5*R*)-2,2-dimethyl-5-tetradecyl-1,3-dioxolan-4-yl)-*N*²-((1'*S*,4'*S*,5'*S*,6'*S*)-4',5'-isopropylidenedioxy-3'-hydroxycyclohexenyl)ethane-1,2-diamine (78% yield); 3) acylation with cerotic acid of the primary amine following the procedure described in example 21 to give (2*S*,3*S*,4*R*)-(3,4-isopropyhdenedioxy)-1-(1'*S*,4'*S*,5'*S*,6'*S*)-4',5'-isopropylidenedioxy-3'-hydroxycyclohexenylaminooctadecan-2-yl)hexacosanamide with a 75% yield; and 4) deprotection of the hydroxylated substituents by reaction with methanol and hydrochloric acid following the next procedure.

To a solution of the amide obtained in step 3 (18 mg, 0.02 mmol) in 10 mL of CH₃OH a drop of HCl (36% in water) is added, and it is stirred for 24 h. The solvents are evaporated to give an oil that solidifies slowly (30 mg, 92 %).

[α]_{D} = +4 (MeOH, C, 0.5); IR (film, cm⁻¹): 3427 (br), 2973, 1659, 994. 1H-NMR (DMSO, 500 MHz, 70°C, mixture of rotamers and conformers); δ, 5.80 (d, J = 10.5, 1H), 5.70 (d, J = 10.5, 1H); 4,85 (m, 1H); 4.23 (m, 2H), 3.95 (m, 2H); 3.82 (m, 2H), 3.40 (m 2H); 2.37 (m, 2H); 1.55 (m, 4H), 1.24 (m, 68H); 0.85 (m, 6H). 13C-NMR (DMSO, 100 MHz, 60°C, mixture of rotamers and conformers): δ 172.0, 134.6, 119.4, 74.9, 70.5, 69.9, 65.8, 64.2, 55.3, 47.4, 43.3, 35.3, 33.7, 30.9, 28.6 (30 C), 24.7, 24.0, 21.7 (2C), 13.3 (2C). HRMS. Calculated for C₅₀H₉₉N₂O₆ (M+H⁺): 823,7503. Found: 823.7546.

### Example 27

### (2S,3S,4R)-2-Hexacosanamide-1-(1'S,4'S,5'S,6'S)-4',5',6'-trihydroxycyclohexylamino)octadecane-3,4-diol.

It is prepared by catalytic hydrogenation with 5% Pd/C (5 mg) that is added to a solution of 5 mg of (2*S*,3*S*,4*R*)-2-hexacosanamide-1-(1'*S*,4'*S*,5'*S*,6'*S*)-4',5',6'-trihydroxycyclohexenylamino)octadecane-3,4-diol in 5 mL of methanol. The mixture is stirred under 1 atm of hydrogen for 24 hours at room temperature. The catalyst is filtered off and it is concentrated to give the compound with a quantitative yield.

[α]_{D} = -5 (MeOH, C, 0.5); IR (film, cm⁻¹); 3429 (br), 2859, 1670, 978. 1H-NMR (DMSO, 500 MHz, 60°C, mixture of rotamers and conformers); δ 8.90 (br, 3H), 8.17 (br, 1H), 4.20-3.08 (m, 8H), 2.32 (m, 2H); 214 (m, 1H), 1.80-1.22 (m, 75H); 0.84 (m, 6H). 13C-NMR (DMSO, 100 MHz, 60°C, mixture of rotamers and conformers): δ 173.0, 75.8, 72.5, 71.6, 68.3, 66.8, 57.2, 50.5, 44.5, 36.2, 34.7, 32.9, 32.0, 30.9, 29.7 (30C), 26.6, 25.0, 22.7 (2C), 14.3 (2C). HRMS. Calculated for C₆₀H₁₀₀N₂O₆ (M+H⁺): 825,7660. Found: 823,7624.

### Example 28:

### (2S,3S,4R)-2-hexacosanamide-1-((1'S,2'S,3'S,4'S,5'S,6'R)-5'-hydroxymethyl-2',3',4',6'-tetrahydroxycyclohexylamino)octadecane-3,4-diol;

This compound was prepared in four steps:
1) formation of the amine following the method described in example 17 by reaction of (1*S*,2*S*,3*S*,4*S*,5*S*,6*R*)-5-hydroxymethyl-2,3,4,5-tetrakis(benzyloxy)cyclohexylamine with (*S*)-2-((4*S*,5*R*)-2,2-dimethyl-5-tetradecyl-1,3-dioxolan-4-yl)-1-(2-nitrophenylsulfonyl)aziridine [Y. Harrak et al. Eur J Org. Chem 2008, 4647-4654] to give the corresponding amine (83% yield);
2) deprotection of the nitrobenzenesulfonyl group according to the procedure described in example 19 to give de compound (*S*)-1-((4*S*,5*R*)-2,2-dimethyl-5-tetradecyl-1,3-dioxolan-4-yl)-*N*²-((1'*S*,2'*S*,3'*S*,4'*S*,5'*S*,6'*R*)-5'-hydroxymethyl-2',3',4',6'-tetrakis(benzyloxy))ethane-1,2-diamine (79% yield);
3) acylation with cerotic acid of the primary amine in said diamine following a procedure analogous to that described in example 21 to give the corresponding (2*S*,3*S*,4*R*)-(3,4-isopropylidenedioxy-1-(1'*S*,2'*S*,3'*S*,4'*S*,5'*S*,6'*R*)-5'-hydroxymethyl-2,3,4,6-tetrabenzyloxycyclohexylaminooctadecan-2-yl)hexacosanamide with a 79% yield;
and 4) deprotection of the hydroxylated substituents of said amide by catalytic hydrogenation following the next procedure. A solution of (0.02 mmol) in 10 mL of CH₃OH containing two drops of conc. HCl is stirred under H₂ (2 atm) in the presence of 10 mg of 5% Pd-C for 48 h. The reaction mixture is filtered and concentrated at low pressure to give the (2*S*,3*S*,4*R*)-2-hexacosanamide-1-((1'*S*,2'*S*,3'*S*,4'*S*,5'*S*,6'*R*)-5-hydroxymethyl-2,'3',4',6'-tetrahydroxycyclohexylamine)octadecane-3,4-diol hydrochloride in the form of a slightly brown solid with an 85% yield.

Pf = 277-279, [α]_{D} = +3 (MeOH, C, 0.5); IR (film): 34000-3200 (br), 2861, 1672, 985. 1H-NMR (CD3OD, 500 MHz); δ 4.36 (m, 1H); 4.26 (m, 1H), 4.14 (m, 3N); 4.00 (m, 2H); 3.92-3.57 m (m, 3H); 3.44 (dd, J = 12.5, 6.5, 1H); 3.26 (dd, J = 12.5, 6, 1H); 2.45 (m, 1H); 2.40 (br, 1H); 2.26 (m, 2H); 1.63 (m, 4H); 1.23 (m, 68H); 0.90 (m, 6H). 13C-NMR (DMSO, 100 MHz, 60 °C): δ 173.3, 75.6, 71.7, 71.4, 67.1, 66.3 (2C), 59.5, 57.1, 46.5, 45.6, 41.8, 35.7, 31.4(2C), 29.7-28.8 (29C), 25.5, 25.3, 22.1 (2C), 13.5, 13.4, HRMS. Calculated for C₅₁H₁₀₂N₂O₈ (M+H⁺): 871.7714. Found: 871.7713.

### Example 29:

### (2S,3S,4R)-2-hexacosanamide-1-((1'S,2'S,3'S,4'S,5'R)-5-hydroxymethyl-2',3',4'-trihydroxycyclohexylamino)octadecane-3,4-diol;

This compound was prepared in four steps:
1) following the method described in example 17 by reaction of (1*S*,2*S*,3*S*,4*S*,5*R*)-5-hydroxymethyl-2,3,4-tris(benzyloxy)cyclohexylamine with (*S*)-2-((4*S*,5*R*)-2,2-dimethyl-5-tetradecyl-1,3-dioxolan-4-yl)-1-(2-nitrophenylsulfonyl)aziridine [Y. Harrak et al. Eur J Org. Chem 2008, 4647-4654] to give the corresponding amine (88% yield);
2) deprotection of the nitrobenzenesulfonyl group according to the procedure described in example 19 to give de compound (*S*)-1-((4*S*,5*R*)-2,2-dimethyl-5-tetradecyl-1,3-dioxolan-4-yl)-*N*²-((1'*S*,2'*S*,3'*S*,4'*S*,5'*R*)-5'-hydroxymethyl-2',3',4'-tris(benzyloxy))ethane-1,2-diamine (69% yield);
3) acylation with cerotic acid of the primary amine following the procedure described in example 21 to give (2*S*,3*S*,4*R*)-(3,4-isopropylidenedioxy-1-(1'*S*,2'*S*,3'*S*,4'*S*,5'*R*)-5'-hydroxymethyl-2',3',4'-tribenzyloxycyclohexylaminooctadecan-2-yl)hexacosanamide with a 74% yield;
and 4) deprotection of the hydroxylated substituents by reaction with methanol and hydrochloric acid following the next procedure: a solution of (0.02 mmol) in 10 mL of CH₃OH containing two drops of conc. HCl is stirred under H₂ (2 atm) in the presence of 10 mg of 5% Pd-C for 48 h. The reaction mixture is filtered and concentrated *in vacuo* to give the (2*S*,3*S*,4*R*)-2-hexacosanamide-1-((1'*S*,2'*S*,3'*S*,4'*S*,5'*R*)-5-hydroxymethyl-2',3',4'-trihydroxycyclohexylamino)octadecane-3,4-diol hydrochloride in the form of a slightly brown solid with an 88% yield.

IR (film, cm⁻¹): 3300-3400 (br), 2966, 2851, 1659, 1440, 1024. 1H-NMR (DMSO, 400 MHz); δ 4.8 (br, 1H); 4.22 (br, 1H); 3.85-3.15 (m, 10H); 2.44 (m, 2H); 2.15 (m, 1H); 2.0 (m, 1H); 1.7 (m, 1H); 1.5 (m, 2H); 1.23 (m, 70H); 0.84 (m, 6H). 13C-NMR (DMSO, 100 MHz, 60 °C): δ 174.9, 76.3, 73.4, 71.8, 68.9, 68.8, 61.4, 58.2, 48.4, 38.7, 36.5, 33.3, 31.8(2C), 29.7-29.0 (30 C), 25.8, 25.1, 22.7 (2C), 14.2 (2C). HRMS. Calculated for C₅₁H₁₀₂N₂O₇ (M+H⁺): 871.7765. Found: 871.7742.

### Example 30:

### (2S,3S,4R)-2-hexacosanamide-1-((1'R,2'S,3'S,4'S,5'R)-5-hydroxymethyl-2',3',4'-trihydroxycyclohexylamino)octadecane-3,4-diol.

This compound was prepared in four steps:
1) following the method described in example 17 by reaction of (1*R*,2*S*,3*S*,4*S*,5*R*)-5-hydroxymethyl-2,3,4-tris(benzyloxy)cyclohexylamine with (*S*)-2-((4*S*,5*R*)-2,2-dimethyl-5-tetradecyl-1,3-dioxolan-4-yl)-1-(2-nitrophenylsulfonyl)aziridine (cfr. Y. Harrak et al., Eur J Org. Chem 2008, 4647-4654) to give the corresponding amine (88% yield);
2) deprotection of the nitrobenzenesulfonyl group according to the procedure described in example 19 to give de compound (*S*)-1-((4*S*,5*R*)-2,2-dimethyl-5-tetradecyl-1,3-dioxolan-4-yl)-*N*²-((1'*R*,2'*S*,3'*S*,4'*S*,5'*R*)-5'-hydroxymethyl-2',3',4'-tris(benzyloxy))ethane-1,2-diamine (69% yield);
3) acylation with cerotic acid of the primary amine following the procedure described in example 21 to give (2*S*,3*S*,4*R*)-(3,4-isopropylidenedioxy-1-(1'*R*,2'*S*,3'*S*,4'*S*,5'*R*)-5'-hydroxymethyl-2,3,4-tribenzyloxycyclohexylaminooctadecan-2-yl)hexacosanamide with a 74% yield;
and 4) deprotection of the hydroxylated substituents by reaction with methanol and hydrochloric acid following the next procedure: a solution of 22.5 mg (0.018 mmol) of the amide in 10 mL of CH₃OH containing two drops of conc. HCl is stirred under H₂ (2 atm) in the presence of 10 mg of 5% Pd-C for 48 h. The reaction mixture is filtered and concentrated *in vacuo* to give 14 mg (0.015 mmol) of the (2*S*,3*S*,4*R*)-2-hexacosanamide-1-((1'*R*,2'*S*,3'*S*,4'*S*,5'*R*)-5-hydroxymethyl-2',3',4'-trihydroxycyclohexylamino)octadecane-3,4-diol hydrochloride in the form of a slightly brown solid with an 81% yield.

IR (neat, cm⁻¹): 3381 (br), 2963, 2841, 1655, 1439, 1024. 1H-NMR (DMSO, 400 MHz); δ 4.83 (br, 1H); 4.23 (br, 1H); 3.85-3.17 (m, 10H); 2.42 (m, 2H); 2.13 (m, 1H); 2.00 (m, 1H); 1.71 (m, 1H); 1.51 (m, 2H); 1.23 (m, 70H); 0.84 (m, 6H). ¹³C-NMR (DMSO, 100 MHz, 60°C): δ 173.9, 76.1, 73.4, 71.7, 68.9, 68.7, 61.6, 58.0, 48.3, 38.6, 36.1, 33.1, 31.9(2C), 29.7-29.3 (30C), 25.9, 25.1, 22.6 (2C), 14.4 (2C). HRMS. Calculated for C₅₁H₁₀₂N₂O₇ (M+H⁺): 871,7765. Found: 871.7742

### Biological assays of the compounds of formula (I)

### Example 31

### Determination of the mouse iNKT cells expansion induced by the aminocyclitol-type compounds in vitro.

The determination of the ability to induce the activation of iNKT cells by the compounds of the invention was performed according to the methods known by the skilled in the art.

First, the ability to induce the specific proliferation of mouse iNKT was determined by culture experiments *in vitro.* For that, spleen cells derived from female B57B1/6 mice between 10 and 20 weeks were isolated and the red blood cells were removed by hypotonic lysis. The spleen cells are distributed in 96 U-well plates, between 100,000 and 500,000 cells per well, and incubated in the presence of each of the compounds 1a, 1b, 2b, 3a, 3b and 4b at a final concentration of 1 µg/mL in RPMI-1640 growth medium, 10% fetal serum, 100 mM glutamine, 1% methanol. At day 2 of culture recombinant human interleukin 2 was added, at a final concentration of 25 units/mL, conditions that did not induce the nonspecific proliferation of NK cells. The culture was analyzed at day 5 or 6 by flow cytometry, using TCR and NK1.1 specific antibodies. In the presence of the control αGalGer: 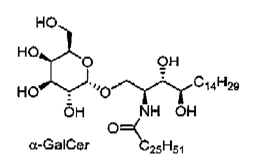 the population of NKT cells was expanded more than 5 times over the baseline conditions, consistent with the results established in the scientific bibliography. In the same conditions, the compound 4b induces a strong proliferation of iNKTs, similar to that of aGalCer, representing an increase of 1,2 to 6,2% of the T cells (fig 1). Likewise, the compounds 3a and 3b, induce a weak expansion of iNKTs (approximately 2 times), but the results are too small to attribute unequivocally an immunostimulating ability. The use of dimethyl sulfoxide (DMSO) instead of methanol as solvent, or the use of higher concentrations of the compounds, only allow to confirm the stimulating trend on iNKTs, but the experiments show too high variability so as to draw definitive conclusions, due to the low solubility of the compounds.

### Quantification of the iNKT cells stimulating ability of the compound 4b

The CD1d glycolipidic ligands induce a deviation in the production of cytokines by iNKT cells to Th1 or Th2, depending on its ability to be presented by CD1d, and its structural characteristics recognized by the TCR. For quantifying more precisely the immunostimulating ability of the compound 4b and determining if it induces a deviation in the iNKT response, its ability to induce the production of IFNγ and IL-4 is titrated in the cell cultures using different concentrations, and comparing the same with the two prototypical ligands, αGalCer and OCH (dissolved in MeOH), that have been described as inducers of a Th1 and Th2 response, respectively.

The compound 4b induces the production of IFN_{γ} at a concentration of 333 ng/mL, reaching the maximal response plateau at 1 µg/mL, this being of lower magnitude and at a higher concentration of compound than in the case of the response produced by α-GalCer (fig. 3a). α-GalCer is represented as aGC in figures 2 and 3. The compound 4b induces a higher relative production of IL-4 versus IFNγ, compared to that induced by aGalCer (IFNγ/IL-4 ratio of 2 in the case of compound 4b versus a ratio of 10 in the case of α-GalCer). This ability of induction of IL-4 is maintained with high efficiency at a concentration of 100 ng/mL, still being significant at 33 ng/mL (fig 3b). Comparing with the Th2 response prototype, OCH, the compound 4b induces a lower response, but retains a similar profile, such that both compounds maintain a much higher ability of IL-4 induction versus IFNγ at lower doses, in contrast with the high Th1 profile of the α-GalCer. Thus, the compound 4b induces preferably Th2-type response after being recognized by iNKTs.

### Cell cultures

Mouse C57BU6 spleen cells are obtained by isolation of the spleen of female mice from 8 to 12 weeks of age, according to protocols approved by the Animal and Human Research Ethics Committee of the Autonomous University of Barcelona. The spleens are disaggregated with the plunger of a syringe in 60 mL plates in a laminar flow hood, removing the red blood cells with lysis buffer (Sigma-Aldrich), 5×10⁵ cells are placed in the wells of 96 U-well plates, and incubated with 100 ng/mL of α-Galcer, OCH, or 1 µg/mL of the compounds 1a, 1b, 2b, 3a, 3b, and 4b in RPMI-1640 medium supplemented with 10% fetal calf serum (FCS) (Labclinics), 50 µM 2-mercaptoethanol, 2mM L-glutamine and 1% methanol (non-toxic concentration for the cells), and grown at 37°C in a 5% CO₂ humidified atmosphere. 25 U/mL of recombinant human interleukin 2 are added at day 2 of culture.

For the determinations of IL-4 and IFNγ, culture supernatants are taken at day 4 or day 7, and stored at -70°C or used immediately in ELISA assays (eBioscience) for quantifying the cytokines, following the manufacturer's instructions. Standard curves are generated with the recombinant cytokines included in the kit. The statistical significance of the results is analyzed using a t-Student test, considering significant differences with p<0.01. The compounds 1a, 1b, 2b, 3a, 3b, and 4b are resuspended in 100% methanol or 100% dimethyl sulfoxide at a concentration of 1 mg/mL, and a 1/10 use dilution in PBS is prepared, with a final concentration of 100 µg/mL. The compounds are heated at 56°C for 10-30 min and sonicated, before being diluted in complete growth medium, with a final concentration of 1% vehicle in the cellular assay.

### Analysis by flow cytometry

The spleen cells cultures grown in the indicated conditions are analyzed by flow cytometry in a FACS Calibur (Beckton Dickinson Bioscience) for quantifying the levels of proliferation of the iNKTs after incubation with the compounds 1a, 1b, 2b, 3a, 3b, and 4b. The stimulated spleen cultures are washed and preincubated with 50 µl of staining medium (PBS with 2% FCS) with anti-CD16 (clone 2.4G2) for 20 min in ice. Subsequently the cells are washed and resuspended in staining medium with mouse anti-TCR antibody conjugated to fluorescein isothiocyanate (FITC) (clone H57-597, BD-Pharmingen) and anti-NK1.1 conjugated to avidin (clone PK-136, BD-Pharmingen) for 30 min in ice. The cells are washed and resuspended in staining medium with streptavidin-phycoerythrin (Southern Biotechonology) for 30 min in ice, The cells are washed twice and resuspended in staining medium. The samples are analyzed in the flow cytometer (FACSCalibur) and the data are processed using the program CeiiQuest (BD Bioscience).

## Claims

1. A compound of general formula (1): 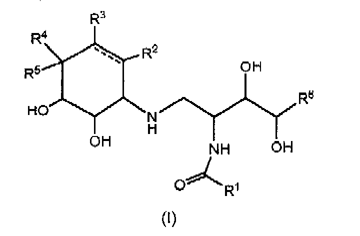 wherein: R¹ is a (C₅-C₃₅)alkyl group, substituted or unsubstituted. R², R³, R⁴ and R⁵ are the same or different from each other, and are selected from the list comprising hydrogen, hydroxyl, alkoxyl or (C₁-C₆)alkyl, substituted or unsubstituted;
R⁶ is selected from the list comprising a (C₅-C₃₅)alkyl, aryl, cycloalkyl, heterocycle; and
---- represents the existence or not of a double bond.
or an isomer, its salts and/or solvate thereof.

2. Compound according to claim 1, wherein R¹ is a (C₇-C₂₅)alkyl group.

3. Compound according to any of claims 1 or 2, wherein R², R³, R⁴ and/or R⁵ are hydroxyl,

4. Compound according to any of claims 1 to 3, wherein R⁴ is hydroxyl and/or R⁵ is hydrogen.

5. Compound according to any of claims 1 to 4, wherein R² is hydrogen or alkoxyl.

6. Compound according to claim 5, wherein R² is methoxyl.

7. Compound according to any of claims 1 to 6, wherein R³ is hydroxyl or a (C₁-C₃)hydroxyalkyl.

8. Compound according to claim 7, wherein R³ is hydroxymethyl.

9. Compound according to any of claims 1 to 8, wherein R³ is a (C₁₀-C₂₀)alkyl group.

10. Compound according to claim 1, of formula:
(2*S*,3*S*,4*R*)-2-octanamide-1-(1'*rs*,2'*RS*,3'*SR*,4'*SR*,5'*RS*,6'*SR*)-2',3',4',5',6'-pentahydroxycyclohexylaminooctadecane-3,4-diol;
(2*S*,3*S*,4*R*)-2-Hexacosanamide-1-(1'*rs*,2'*RS*,3'*SR*,4'*sr*,5'*RS*,6'*SR*)-2',3',4',5',6'-pentahydroxycyclohexylaminooctadecane-3,4-diol;
(2*S*,3*S*,4*R*)-2-octanamide-1-(1'*R*,2'*S*,3'*R*,4'*S*,5'*S*,6'*S*)-2',3',4',5',6'-pentahydroxycyclohexylaminooetadecane-3,4-diol;
(2*S*,3*S*,4*R*)-2-Hexacosanamide-1-(1'*R*,2'*S*,3'*R*,4'*S*,5'*S*,6'*S*)-2,3,4,5,6-pentahydroxycyclohexylaminooctadecane-3,4-diol;
(2*S*,3*S*,4*R*)-2-hexacosanamide-1-(1'*S*,2'*S*,3'*R*,4'*R*,5'*S*)-2,3,4,5,-tetrahydroxycyclohexylaminooctadecane-3,4-diol;
(2*S*,3*S*,4*R*)-2-hexacosanamide-1-(1'*R*,2'*S*,3'*R*,4'*R*,5'*S*)-2,3,4,5,-tetrahydroxycyclohexylaminooctadecane-3,4-diol;
(2*S*,3*S*,4*R*)-2-hexacosanamide-1-(1'*S*,2'*S*,3'*R*,4'*R*,5'*S*,6'*S*)-2,3,4,5,-tetrahydroxy-6-methoxycyclohexylaminooctadecane-3,4-diol;
(2*S*,3*S*,4*R*)-2-Hexacosanamide-1-(1'*S*,4'*S*,5'*S*,6'*S*)4',5',6'-trihydroxycyclohexenylaminooctadecane-3,4-diol;
(2*S*,3*S*,4*R*)-2-Hexacosanamide-1-(1'*S*,4'*S*,5'*S*,6'*S*)-4',5',6'-trihydroxycyclohexylaminooctadecane-3,4-diol;
(2*S*,3*S*,4*R*)-2-hexacosanamide-1-((1'*S*,2'*S*,3'*S*,4'*S*,5'*S*,6'*R*)-5-hydroxymethyl-2,3,4,6,-tetrahydroxycyclohexylamino)octadecane-3,4-diol;
(2*S*,3*S*,4*R*)-2-hexacosanamide-1-((1'*S*,2'*S*,3'*S*,4'*S*,5'*R*)-5-hydroxymethyl-2,3,4,-trihydroxycyclohexylamino)octadecane-3,4-diol; or
(2*S*,3*S*,4*R*)-2-hexacosanamide-1-((1'*R*,2'*S*,3'*S*,4'*S*,5'*R*)-5-hydroxymethyl-2,3,4,-trihydroxycyclohexylamino)octadecane-3,4-diol.

11. Compound according to any of claims 1 to 10, wherein said compound is a hydrochloride salt.

12. Process for obtaining the compound of general formula (I) comprising:
- the coupling of an aminocyclitol of general formula (II) 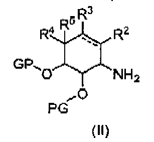 with an aziridine of general formula (V) by nucleophilic attack, 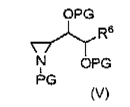 or with an aldehyde of general formula (VI) by reductive amination: 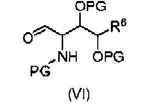 to give the intermediate compounds (VII), 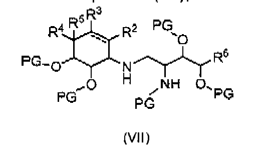
- and the subsequent removal of the protecting groups PG and acylation,
wherein: R¹, R², R³, R⁴, R⁵ and R⁶ are as described in claim 1 and PG is a protecting group.

13. Process according to claim 12, wherein the aminocyclitol of general formula (II) is obtained:
- by reduction of the compound of general formula (III); 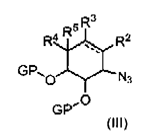
- or by substitution of the compounds of general formula (IV) 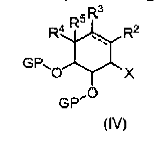
wherein: R¹, R², R³, R⁴ and R⁵ are as described in claim 1, X is halide or sulfonate and PG is a protecting group.

14. Use of the compound of general formula (I) for the preparation of a pharmaceutical composition.

15. Use of the compound of general formula (I) for the preparation of a pharmaceutical composition for the treatment and/or prevention of diseases through the stimulation of iNKT cells.

16. Use according to claim 15, wherein the treatable or preventable diseases through the stimulation of iNKT cells are selected from the list comprising: autoimmune diseases, cancer, microbial infections or inflammatory diseases.

17. Use according to claim 16, wherein the autoimmune diseases are selected from the list comprising asthma, COPD, chronic colitis, several allergies, systemic lupus erythematosus, type 1 diabetes mellitus, multiple sclerosis, Sjögren syndrome or rheumatoid arthritis.

18. Use according to claim 16, wherein the infections caused by pathogenic microorganisms are selected from the list comprising flu, AIDS, hepatitis, chlamydiosis, leishmaniasis, malaria, tuberculosis, trypanosomiasis, streptococcosis, or pseudomoniasis.

19. Pharmaceutical composition comprising at least a compound of general formula (I) and a pharmaceutically acceptable vehicle.

20. Pharmaceutical composition according to claim 19 further comprising another active ingredient.
